# EUROPÄISCHE PATENTSCHRIFT

(11) **EP 1 138 347 B1**
(45) Veröffentlichungstag und Bekanntmachung des Hinweises auf die Patenterteilung: **25.10.2006**
(21) Anmeldenummer: 01250109.4
(22) Anmeldetag: 29.03.2001
(51) Int. Cl.: A61N 1/37, A61N 1/39, A61N 1/362

(54) **Vorrichtung zur Verarbeitung physiologischer Signale**
Device for processing physiological signals
Dispositif de traitement de signaux physiologiques

(30) Priorität: 01.04.2000 DE 10017620
(43) Veröffentlichungstag der Anmeldung: 04.10.2001
(73) Patentinhaber: BIOTRONIK GmbH & Co. KG, 12359 Berlin (DE)
(72) Erfinder: Schomburg, Richard A., Hillsboro, Oregon 97123-9048 (US); Bauer, Peter Thomas, West Linn, Oregon 97068 (US)
(74) Vertreter: Lindner-Vogt, Karin L.

(56) Entgegenhaltungen:
- EP-A- 0 738 522
- US-A- 4 750 494

## Beschreibung

Die Erfindung betrifft eine Vorrichtung zur Verarbeitung physiologischer Signale, welche eine Signalaufnahmeeinheit zur Aufnahme physiologischer Meßsignale, insbesondere zur Aufnahme elektrischer Signale eines Herzen umfaßt, sowie erste Detektionsmittel, die mit der Signalaufnahmeeinheit verbunden sind und Signalbearbeitungsmittel, wie einen Filter sowie einen ersten Schwellwertspeicher und eine mit diesem verbundene Vergleichseinheit umfassen, die zum Ausgeben eines ersten Markersignals ausgebildet ist, falls die Amplitude des Meßsignals einen im ersten Schwellwertspeicher gespeicherten Schwellwert überschreitet, und zweite Detektionsmitteln, die mit der Signalaufnahmeeinheit verbunden sind, und einen zweiten Schwellwertspeicher sowie eine mit diesem verbundene zweite Vergleichseinheit umfassen, die zum Ausgeben eines zweiten Markersignals ausgebildet ist, falls die Amplitude des Meßsignals einem im zweiten Schwellwertspeicher gespeicherten Schwellwert überschreitet.

Derartige Signalverarbeitungsvorrichtungen können beispielsweise Bestandteil eines Herzschrittmachers, eines Kardioverters und/oder eines Defibrillators sein. Ein spezieller Zweck solcher Signalverarbeitungsvorrichtungen ist das Erkennen von Kammerflimmern, insbesondere das Erkennen ventrikulärer Fibrillationen, die eine Antitachykardietherapie oder eine Kardioversion erforderlich machen.

Insbesondere das Detektieren ventrikulärer Fibrillationen, daß heißt physiologisch unangemessen hoher Herzraten, ist mit einigen Problemen verbunden. Es ist grundsätzlich bekannt, die Herzraten anhand eines Elektrokardiogramms zu bestimmen, in dem die R-Zacken die in dem Elektrokardiogramm enthaltenen QRS-Komplexe detektiert werden und die Frequenz, mit der die R-Zacken auftreten, die RR-Rate bestimmt wird. Problematisch ist hierbei vor allem eine stark wechselnde Amplitude der R-Zacken eines Elektrokardiogramms und die unteschiedliche Breite der QRS-Komplexe.

Aus dem Stand der Technik sind verschiedene Vorrichtungen zur Erfassung ventrikulärer Fibrillationen bekannt. In der US 4,393,877 wird vorgeschlagen, zwei Eingangskanäle für die Meßsignalerfassung vorzusehen, die eine unterschiedliche Empfindlichkeit für die Anstiegssteilheit von EKG-Signalen besitzen und über die Anstiegssteilheit erkannte Signalmerkmale der beiden Eingangskanäle über eine "exklusiv-Oder"-Funktion zu verknüpfen. In der US Patentschrift 4,880,004 ist ein Herzstimulator beschrieben, der zwei Eingangskanäle mit unterschiedlicher Bandpass-Charakteristik besitzt, von denen einer zur Detektion kardialer Ereignisse dient und von denen der zweite der automatischen Anpassung der Detektions-Empfindlichkeit des ersten Eingangskanals dient.

Die US Patentschrift 5,562,709 betrifft einen atrialen Defibrillator, der zwei Eingangskanäle zur R-Zacken Detektion besitzt, welche unterschliedliche Bandpass-Charakteristiken aufweisen und mit jeweils eigenen Amplitudenschwellwerten für die R-Zacken-Detektion arbeiten. Die Detektionsergebnisse beider Eigangskanäle werden zum Erzeugen eines Synchonisationssignals logisch "Und"-verknüpft. Zum Zurücksetzen eines Intervalltimers werden die Detektionsergebnisse beider Eigangskanäle logisch "Oder"-verknüpft. Für das Detektieren von atrialen Fibrillationen ist ein dritter, separaterEingangskanal vorgesehen.

Die US Patentschrift US 4,750,494 betrifft eine Vorrichtung zur Detektion von ventrikularen Fibrillationen, welche Vorrichtung zwei Detektionsmittel aufweist.

Es hat sich gezeigt, daß die mit Hilfe der bekannten Vorrichtungen mögliche Detektion insbesondere ventrikulärer Fibrillationen vor allem hinsichtlich der Zuverlässigkeit Wünsche offen läßt.

Ziel der vorliegenden Erfindung ist es daher, eine Vorrichtung zur Verarbeitung physiologischer Signale anzugeben, die mit möglichst einfachen Mitteln eine sichere Detektion von insbesondere in Herzsignalen enthaltenen Ereignissen und/oder bestimmte Zustände des Herzens, wie das Vorliegen einer ventrikulären Fibrillation, erlaubt.

Erfindungsgemäß wird dieses Ziel mit Hilfe einer Vorrichtung der eingangs genannten Art erreicht, welche eine Auswerteeinheit umfaßt, die mit dem ersten und dem zweiten Detektionsmitteln zur Übernahme jeweils einer Folge der ersten und der zweiten Markersignale verbunden und zur Verarbeitung der Folgen von Markersignalen zu einer einzigen Folge von zusammengefaßten Markersignalen, zur Analyse der zeitlichen Abfolge der Markersignale, insbesondere der Intervalle zwischen Markersignalen der zusammengefaßten Folge und Ausgabe eines von der Analyse abhängigen Erkennungssignals ausgebildet ist.

Im Kontext der Erfindung ist das Erkennungssignal ein Signal, welches einen zu detektierenden Zustand eines Herzens bezeichnet und nicht ein Ereignis wie eine R-Zacke als solche. Die Analyse der zusammengefaßten Folge ist somit nicht auf die jeweils einzelnen Ereignisse wie R-Zacken gerichtet, sondern auf die durch die Markersignale repräsentierte zeitliche Abfolge von Ereignissen, insbesondere die Intervalle zwischen den Markersignalen als bereits gefilterte Wiedergabe der Ereignisse. Diese Analyse ist auf das Detektieren solcher Zustände wie Fibrillationen angelegt. Eine derartige Vorrichtung ermöglicht es in vorteilhafter Weise, RR-Intervalle und damit Herzraten durch Analyse der Intervalle zwischen Markersignalen einer zusammengefaßten Folge von Markersignalen zu bestimmen, wobei die zusammengefaßte Folge Markersignale enthält, die von Eingangskanälen mit unterschiedlichen Detektionseigenschaften stammen. Insbesondere erlaubt es eine derartige Vorrichtung, einerseits die für die Detektion von Ereignissen und Ausgabe entsprechender Markersignale entscheidenenden Parameter der Detektionsmittel, wie Eingangsempfindlichkeit, Frequenzgang und Größe des Schwellwertes, sowie andererseits die Art und Weise, wie die Folgen auf zwei unterschiedliche Weise gewonnener Markersignale durch entsprechende Mittel zu einer Folge zusammengefaßt werden, so zu wählen und aufeinander abzustimmen, daß die Erkennung von Herzraten und damit auch die Fibrillationserkennung zuverlässig erfolgen kann.

Zur Analyse der zusammengefaßten Folge von Markersignalen umfaßt die Auswerteinheit vorzugsweise einen Zähler, der zum Zählen der Markersignale oder der Intervalle zwischen Markersignalen innerhalb eines vorgegebenen Abschitts der gefilterten Folge von Markersignalen ausgebildet ist. Dies alleine erlaubt bereits die Bestimmung der Herzrate für den jeweiligen Meßsignalabschnitt. In Kombination mit der Analyse der Intervalllängen innerhalb des jeweiligen Abschnitts der zusammengesetzten Folge von Markersignalen läßt sich ein zuverlässiges Kriterium für das Vorliegen einer Fibrillation bilden, wie im folgenden detailierter beschrieben ist.

Bevorzugt ist eine Analyse, für die eine bestimmte Anzahl von beispielsweise 48 Intervallen abgezählt und geprüft wird, wieviele dieser 48 Intervalle kürzer sind, als ein ein Vergleichsintervall. Ein eine Fibrillation anzeigendes Erkennungssignal wird dann ausgegeben, wenn 36 der 48 Vergleichsintervalle kürzer sind, als das Vergleichsintervall. Die Auswerteeinheit umfaßt in diesem Fall beispielsweise Mittel zum Zählen einer Gesamtzahl von Intervallen sowie zum Zählen derjenigen Intervalle innerhalb dieser Gesamtzahl von Intervallen, die kürzer sind als ein Vergleichsintervall. Außerdem umfaßt die Auswerteeinheit Mittel zum Vergleichen der gezählten Werte mit entsprechenden Vergleichswerten und Mittel zum Ausgeben des Erkennungssignals in Abhängigkeit des Vergleichsergebnisses.

In einer bevorzugten Ausführungsform der Vorrichtung umfassen die ersten Selektionsmittel eine erste Absolutwerteinheit, die zum Bilden des Absolutwertes des Meßsignales ausgebildet ist. Diese Absolutwerteinheit ist vorzugsweise der ersten Vergleichseinheit vorgeschaltet. Auf das Meßsignal bezogen bedeutet dies, daß negative Meßsignalwerte mit umgekehrten Vorzeichen für den Schwellwertvergleich herangezogen werden, während positive Meßsignalwerte ihr Vorzeichen behalten. Es hat sich gezeigt, daß dies die Zuverlässigkeit der Fibrillationserkennung deutlich erhöht.

Auch die zweiten Detektionsmittel umfassen vorzugsweise eine zweite Absolutwerteinheit, die der zweiten Vergleichseinheit vorgeschaltet ist.

Weiterhin ist vorzugsweise ein Hochpassfilter für das Meßsignal vorgesehen, der entweder bereits in der Signalaufnahmeeinheit angeordnet ist, oder den zweiten Detektionsmitteln zugeordnet, so daß das von den zweiten Detektionsmitteln zu verarbeitende Signal mit einer Grenzfrequenz, die vorzugsweise eine Größenordnung von 10 Hz hat, hochpassgefiltert ist. Auf diese Weise werden nieder frequente Störsignale unterdrückt, ohne die Bandbreite des von den zweiten Detektionsmitteln zu verarbeitenden Signals allzu sehr einzuschränken.

Die zweiten Detektionsmittel weisen vorzugsweise einen Hochpassfilter mit einer Grenzfrequenz auf, die vorzugsweise eine Größenordnung von 20 bis 30 Hz hat und somit über derjenigen des Hochpassfilters in der Signalaufnahmeeinheit oder der ersten Detektionsmittel liegt. Das von den zweiten Detektionsmitteln zu verarbeitende Signal ist damit stärker frequenzbegrenzt, als das von den ersten Detektionsmitteln zu verarbeitende Signal. Solche Meßsignalanteile mit einer Frequenz von 10 bis 20 oder 30 Hz werden damit nur von den ersten Detektionsmitteln verarbeitet.

Weiterhin weisen die ersten Detektionsmittel vorzugsweise eine Rauschbegrenzungseinheit auf, welche die Weiterverarbeitung des Meßsignals innerhalb einer vorgebbaren Rauschunterdrückungszeit nach solchen Signalwerten des Meßsignals unterdrückt, die zu einem ersten Markersignal führen. Jeder solcher Signalwerte löst dabei die erste Rauschunterdrückungszeitdauer erneut aus. Die erste Rauschbegrenzungseinheit ist damit retriggerbar. Die erste Rauschunterdrückungszeitdauer liegt vorzugsweise in der Größenordnung von 125 ms. Ausgelöst wird die erste Rauschunterdrückungszeitdauer vorzugsweise von der ansteigenden Signalflanke desjenigen Signalwertes, der den ersten Schwellwert überschreitet und somit zu einem ersten Markersignal führt. Entsprechend ist ein Flankendetektor für das Detektieren der ansteigenden Signalflanke vorgesehen.

Auch die zweiten Detektionsmittel weisen vorzugsweise eine Rauschbegrenzungseinheit zum Auslösen einer zweiten Rauschunterdrückungszeitdauer auf, die von der ansteigenden Flanke eines solchen Signalwertes ausgelöst wird, der den zweiten Schwellwert überschreitet. Auch die zweiten Detektionsmittel weisen somit vorzugsweise einen Flankendetektor auf. Die ausgelöste Rauschunterdrükkungszeitdauer ist retriggerbar, das heißt sie wird von jedem auch innerhalb der Rauschunterdrückungszeitdauer auftretenden, schwellwertüberschreitenden Signalwerterneut ausgelöst. Die zweite Rauschunterdrückungszeitdauer hat eine Größenordnung von vorzugsweise 50 ms.

Außerdem weisen die ersten Detektionsmittel vorzugsweise einen Refraktärzeitgeber auf, welcher eine erste Refraktärzeit auslöst, falls ein Meßsignalwert den ersten Schwellwert überschreitet und somit ein erstes Markersignal auslöst, sofern dies außerhalb der ersten Refraktärzeit geschieht. Der Refraktärzeitgeber ist somit nicht retriggerbar und spricht nur auf außerhalb der Refraktärzeit liegende Signal an. Die erste Refraktärzeit setzt sich zusammen aus einer absoluten Refraktärzeit von vorzugsweise 150 ms und einer relativen Refraktärzeit, wobei die gesamte Refraktärzeit vorzugsweise 300 ms beträgt. Während der gesamten Refraktärzeit von 300 ms auftretende Ereignisse nach der Detektion eines schwellwertüberschreitenden Signalwertes werden für die weitere Bearbeitung nicht berücksichtigt. Der erste Refraktärzeitgeber ist der ersten Rauschbegrenzungseinheit nachgeschaltet.

Auch die zweiten Detektionsmittel umfassen vorzugsweise einen zweiten, nicht retriggerbaren Refraktärzeitgeber für eine zweite Refraktärzeit. Die zweite Refraktärzeit beträgt vorzugsweise 135 ms.

Die Flankendetektoren zum Detektieren ansteigender Signalflanken und Auslösen der ersten oder zweiten Rauschunterdrückungszeitdauer beziehungsweise der ersten oder zweiten Refraktärzeit sind vorzugsweise so ausgebildet, daß sie auf einen Schwellwertdurchgang des Meßsignales in positiver Richtung ansprechen.

Wesentliches Merkmal der erfindungsgemäßen Vorrichtung ist die Auswerteeinheit; Diese ist zur Übernahme der von den ersten und zweiten Detektionsmittel stammenden Folgen erster und zweiter Markersignale ausgebildet, und zwar derart, daß der über das Meßsignal und seiner schwellwertüberschreitenden, zum Markersignalen führenden Signalwerte gegebene zeitliche Zusammenhang zwischen den ersten und zweiten Markersignalen erhalten bleibt. Die Übernahme der Folgen erster und zweiter Markersignale von den ersten und zweiten Detektionsmittel erfolgt somit zueinander zeitlich korreliert beziehungsweise miteinander synchronisiert.

In einer bevorzugten Ausführungsvariante umfaßt die Auswerteeinheit eine Logikeinheit, durch welche die Folgen erster und zweiter Markersignale nach Art einer "Oder"-Verknüpfung zu einer gemeinsamen Folge zusammengefaßt werden, wobei innerhalb eines Zeitfensters von vorzugsweise + /- 50 ms liegende erste und zweite Markersignale zu einem Markersignal der zusammengefaßten Folge zusammengefaßt werden. Diesezusammengefaßte Folge wird durch eine logische Filtereinheit vorzugsweise derart gefiltert, daß die gefilterte Folge von Markersignalen nur solche Markersignale enthält, die außerhalb einer Ausblendzeit von vorzugsweise 300 ms liegen, welche durch jedes Markersignal der von den zweiten Detektionsmitteln stammenden zweiten Folge von Markersignalen ausgelöst wird.

In einer besonders bevorzugten alternativen Ausführungsform umfaßt die Auswerteeinheit:
- Koinzidenzerkennungsmittel, die ausgebildet sind, ein Koinzidenzsignal auszugeben, wenn eines der ersten Markersignale außerhalb der ersten Refraktärzeit und eines der zweiten Markersignale außerhalb der zweiten Rauschunterdrückungszeitdauer in einen Koinzidenzzeitraum von vorzugsweise 50 ms fallen, sowie
- Ausblendmittel, die mit den Koinzidenzerkennungsmitteln verbunden und ausgebildet sind, solche der zeiten Markersignale für die weitere Signalverarbeitung auszublenden, die innerhalb einer ersten Ausblendzeit von vorzugsweise 300 ms auf ein zweites Markersignal folgen, welches zu einem Koinzidenzsignal geführt hat, sowie
- Koinzidenztransfermittel, die mit den Koinzidehzerkennungsmittein verbunden und ausgebildet sind, das erste der ersten Markersignale, welches außerhalb der ersten Rauschunterdrückungszeitdauer und innerhalb einer Transferzeitdauer von vorzugsweise 300 ms auf ein Koinzidenzsignal folgt, unter Beibehalt des Zeitzusammenhanges der Folge der zweiten Markersignale hinzuzufügen und so eine modifizierte Folge von Markersignalen zu bilden,
- Koinzidenztransferausblendmittel, die mit den Koinzidenztransfermitteln verbunden und ausgebildet sind, aus der modifizierten Folge von Markersignalen all diejenigen Markersignale auszublenden, die innerhalb einer zweiten Ausblendzeit von vorzugsweise 300 ms auf ein von den Koinzidenztransfermitteln hinzugefügtes Markersignal folgen, um so eine gefilterte Folge von Markersignalen zu bilden.

Mit einer derartigen Auswerteeinheit wird eine Reihe von Regeln in die Praxis umgesetzt, die zu einer optimierten zusammengesetzten Folge für die weitere Analyse führen.

Die beiden alternativen Ausführungsvarianten der Auswerteeinheit umfassen vorzugsweise zusätzlich:
- Intervallbestimmungsmittel, welche ausgebildet sind, die Intervalldauer zwischen zwei benachbarten Markersignalen der gefilterten Folge von Markersignalen liegender Zeitintervalle zu bestimmen, sowie einen
- Vergleichsintervallspeicher für eine Vergleichsintervallzeitdauer und
- Intervallvergleichsmittel, die mit den Intervallbestimmungsmitteln sowie dem Vergleichsintervallspeicher verbunden und ausgebildet sind, diejenigen Intervalle der gefilterten Folge von Markersignalen zu detektieren, die kürzer sind, als die Vergleichsintervallzeitdauer,
- einem Zähler, der mit den Intervallvergleichsmitteln verbunden und ausgebildet ist, die innerhalb eines Abschnitts der gefilterten Folge von Markersignalen liegende Anzahl von Markersignalen oder Intervallen zwischen jeweils zwei benachbarten Markersignalen zu bestimmen sowie die Anzahl dieser Intervalle, die kürzer sind, als die Vergleichsintervallzeitdauer, und
- einen Signalgeber, der mit der Intervallzähleinheit und einem Quotientenspeicher verbunden und derart ausgebildet ist, daß der Signalgeber ein Signal ausgibt, falls der Anteil der Intervalle, die kürzer als die Vergleichsintervallzeitdauer sind einen in dem Quotientenspeicher gespeicherten Anteil an der Gesamtzahl von Intervallen eines Abschnittes der gefilterten Folge überschreitet.

Durch diese Mittel ist die Auswerteeinheit in der Lage, eine bestimmte Anzahl von Intervallen abzuzählen und zu bestimmen, wieviele dieser Intervalle kürzer sind, als ein Vergleichsintervall. Konkret kann die Auswerteeinheit so ausgebildet sein, daß der Zähler eine vorgegebene Anzahl von Intervallen abzählt, beispielsweise 48, und mittels der Intervallvergleichsmittel diejenigen Intervalle markiert, die kürzer sind als das Vergleichsintervall. Überschreitet die Anzahl der markierten Intervalle innerhalb der 48 Intervalle beispielsweise den Wert 36 wird ein eine Fibrillation kennzeichnendes Signal ausgegeben. Wenn der Zähler somit so ausgebildet ist, jeweils 48 Intervalle zu zählen, kann der Signalgeber einen zweiten Zähler für die Intervalle enthalten, die kürzer sind als das Vergleichsintervall. In dem Quotienspeicher ist dann ein Grenzwert für die Zahl dieser kürzeren Intervalle gespeichert und das Signal wird ausgelöst, wenn der Wert in dem zweiten Zähler den in dem Quotienten gespeicherten Wert überschreitet. Wenn mit anderen Worten durch den ersten Zähler der Nenner des Quotienten, beispielsweise 48, vorgegeben ist, muß der Quotientenspeicher des Signalgebers nur noch den Zähler des Quotienten, beispielsweise 36, enthalten. Auch andere an sich bekannte Mittel, die dazu führen, daß die Auswerteeinheit ein Signal ausgibt, wenn von beispielsweise 48 zuletzt erfaßten Intervallen mehr als 36 kürzer sind als ein Vergleichsintervall, sind für die Realisierung alternativer Ausführungsvarianten der Auswerteeinheit geeignet.

Die Signalaufnahmeeinheit umfaßt vorzugsweise einen Dezimator, der das Meßsignal mit vorzugsweise 4096 Hz abtastet und in einem Takt von vorzugsweise 512 Hz Abtastwerte für das Meßsignal ausgibt. Dies erflaubt eine Abtastung des Meßsignals mit ausreichender Genauigkeit und führt zu einer ersten Datenreduktion, die den Aufwand für die Weiterbearbeitung des Meßsignals sinnvoll beschränkt.

Außerdem umfaßt die Signalaufnahmeeinheit vorzugsweise einen Analog/Digital-Wandler für das Meßsignal, so daß das Meßsignal in eine Folge zeitdiskrete, digitaler Wert umgewandelt wird, die digital weiterverarbeitet werden kann.

Vorzugsweise umfaßt die Signalaufnahmeeinheit weiterhin Mittelwertbildungsmittel zur Bildung eines gleitenden Mittelwertes des Meßsignals. Hierdurch werden möglicherweise einer Drift unterworfene Gleichsignalanteile des Meßsignals vor der Weiterverarbeitung durch die beiden Detektionsmittel unterdrückt.

Die ersten Detektionsmittel besitzen vorzugsweise einen Signalverstärker für das Meßsignal mit einem anpaßbaren Verstärkungsfaktor. Im Falle eines Herzstimulators wird der Verstärkungsfaktor vorzugsweise dann erhöht, wenn der Stimulator einen Stimulationsimpuls abgibt. Zusätzlich oder alternativ wird der Verstärkungsfaktor auch dann erhöht, wenn man die zweiten Detektionsmittel innerhalb von 200 ms vor der Abgabe des Stimulationsimpulses einen Signalwert detektiert haben, der den zweiten Schwellwert überschreitet und somit ein zweites Markersignal zur Folge hat.

Die zweiten Detektionsmittel sind vorzugsweise abschaltbar ausgeführt und werden durch eine Aktivierungseinheit aktiviert, falls der Verstärkungsfaktor des Signalverstärkers der ersten Detektionsmittel erhöht wird.

Alternativ oder zusätzlich aktiviert die Aktivierungseinheit die zweiten Detektionsmittel auch dann, wenn die von den ersten Detektionsmitteln detektierten Signale eine Frequenz von 140 Signalen pro Minute überschreiten.

Die zweiten Detektionsmittel können zur zeitversetzten Bearbeitung der Meßsignalwerte ausgebildet sein und umfassen dazu vorzugsweise einen Signalspeicher, für das von den Mittelwertbildungsmitteln ausgegebene, digitalsierte Meßsignal. Dieser Signalspeicher kann als Ringspeicher oder First-In-First-Out-Speicher (FIFO-Speicher) ausgebildet ist.

Die Erfindung soll nun anhand eines Ausführungsbeispiels mit Hilfe der Figuren näher erläutert werden. Diese zeigen:
- Figur 1 ein Blockdiagramm einer Ausführungsform einer Signalverarbeitungsvorrichtung;
- Figur 2 eine Detaildarstellung der Auswerteeinheit der Signalverarbeitungsvorrichtung aus Figur 1;
- Figur 3 eine vereinfachte Darstellung der Vorrichtung aus Figur 1 sowie eines Meßsignals und der von den ersten und zweiten Detektionsmitteln ausgegebenen Signale;
- Figur 4 eine Darstellung ähnlich wie Figur 3;
- Figur 5 eine schematische Darstellung zur Erläuterung der Arbeitsweise der Auswerteeinheit;
- Figuren 6 bis 11 verschiedene Meßsignale und die entsprechenden Signale der ersten und zweiten Detektionsmittel.

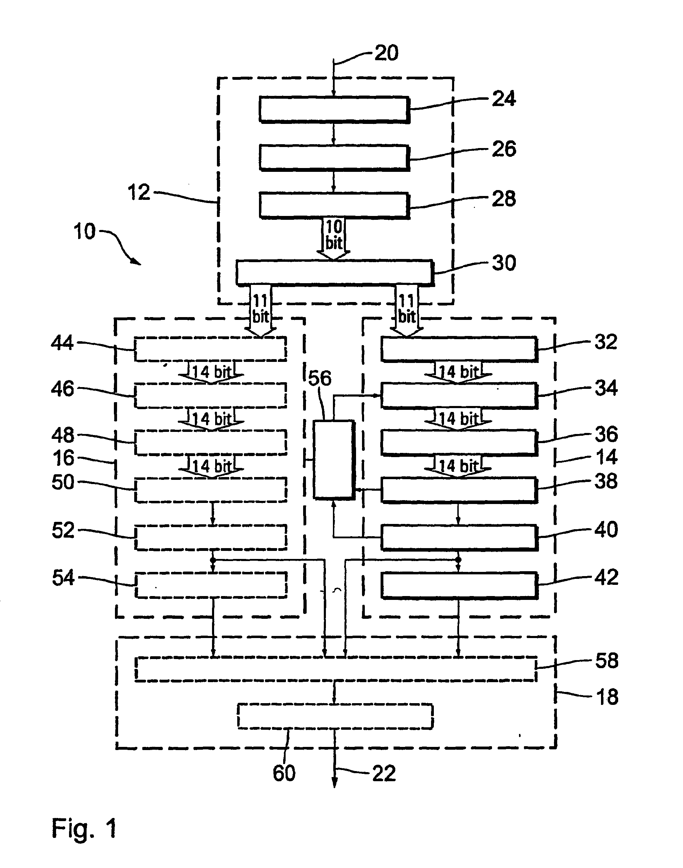

Dem Blockdiagramm der Signalverarbeitungsvorrichtung 10 in Figur 1 ist zu entnehmen, daß die Signalverarbeitungsvorrichtung 10 als vier wesentliche Bestandteile eine Signalaufnahmeeinheit 12, erste Detektionsmittel 14, zweite Detektionsmittel 16 und eine Auswerteeinheit 18 umfaßt. An einem Meßeingang 20 liegt ein elektrisches Signal an, das einem Elektrokardiogramm eines Herzens entspricht. Das Signal kann ein Testsignal sein oder es kann intrakardial aufgenommen sein. Über einen Signalausgang 22 gibt die Signalverarbeitungsvorrichtung 10 dann ein Ausgangssignal ab, wenn die Signalverarbeitungsvorrichtung 10 mittels ihrer zuvor beschriebenen Bestandteile anhand des Meßsignals eine Fibrillation detektiert hat.

Die Signalaufnahmeeinheit 12 weist eingangsseitig den Meßeingang 20 auf und ist ausgangsseitig mit den ersten Detektionsmitteln 14 und den zweiten Detektionsmitteln 16 verbunden. Bestandteile der Signalaufnahmeeinheit 12 sind ein Anti-Aliasing-Filter 24, der mit dem Meßeingang 20 verbunden ist. Ausgangsseitig ist der Anti-Aliasing-Filter mit einem Dezimator 26 verbunden, der seinerseits wiederum mit einem Analog/Digital-Wandler 28 verbunden ist. Bis zum Analog/Digital-Wandler 28 wird das Meßsignal analog verarbeitet. Ausgangsseitig ist der Analog /Digital-Wandler 28 mit einem Mittelwertfilter 30 zur gleitenden Mittelwertbildung verbunden. Der Mittelwertfilter 30 besitzt zwei identische Ausgänge, die zu den ersten Detektionsmitteln 14 einerseits und zu den zweiten Detektionsmitteln 16 andererseits führen.

Der Anti-Aliasing-Filter 24 ist ein passiver, hyprider Bandpass-R-C-Filter mit einem Frequenzgang (-3 dB) von 10 Hz bis 224 Hz. Der Dezimator 26 ist ein Dezimator mit Umschaltkondensatoren mit einer Eingangsabtastrate von 4.096 Hz sowie einer Ausgangsdatenrate von 512 Hz. Er weist eine programmierbare Verstärkung von 20/9 bis 80/9 auf. Jeder Ausgangswert ist die Summe von 8 Eingangstastwerten, multipliziertmitdemVerstärkungsfaktor. DerAnalog/Digital-Wandler 28 ist für eine Eingangsspannung zwischen -500 mV und + 500 mV ausgelegt und bietet ausgangsseitig eine Datenauflösung von 10 bit in Zweierkomplementdarstellung. Der Mittelwertfilter 30 berechnet einen gleitenden Mittelwert derart, daß jeder Ausgangswert die Summe des aktuellen und der vorangehenden Eingangswerte ist. Er besitzt einen Verstärkungsfaktor von 2 und eine Datenauflösung am Ausgang von 11 bit.

Die ersten Detektionsmittel 14 umfassen die folgenden Bestandteile: Einen Hochpassfilter 32, einen programmierbaren Verstärker 34, eine Absolutwerteinheit 36, einen Schwellwertdetektor 38, einen Rauschbegrenzer 40 und einen Refraktärzeitgeber 42. Diese Bestandteile sind in der aufgezählten Reihenfolge hintereinander geschaltet, das heißt der Ausgang eines zuvor genannten Bestandteils ist jeweils mit dem Eingang des nächst genannten Bestandteils verbunden. Der Hochpassfilter 32 ist ein Butterworth-Hochpassfilter 2. Ordnung mit einer Datenauflösung am Ausgang von 14 bit und einer Grenzfrequenz von 18 Hz. Sein Verstärkungsfaktor ist 8. Der programmierbare Verstärker 34 ist hinsichtlich des Verstärkungsfaktors programmierbar. Die Eingangsempfindlichkeit kann in 15 Stufen zwischen 0,5 mV und 7,5 mV eingestellt werden. Bei einer Empfindlichkeit von 5 mV beträgt der Verstärkungsfaktor 2,1875. Die Absolutwerteinheit 36 wandelt ein eingangsseitig zwischen positiven und negativen Werten ein Eingangssignal in ein Ausgangssignal um, dessen Signalwerte den Betrag der Eingangssignalwerte entspricht. Entsprechend werden negative Signalwerte des Eingangssignals in positive Signalwerte gleichen Betrags des Ausgangssignals gewandelt, während positive Eingangssignalwerte unverändert bleiben. Der Schwellwertdetektor 38 gibt ein Signal 1 für jeden Eingangswert aus, der einen gespeicherten Schwellwert von 850 überschreitet. Anderenfalls gibt der Schwellwertdetektor 38 kein Ausgangssignal bzw. logisch "0" aus. Der Rauschbegrenzer 40 blendet alle Signalwerte des Schwellwertdetektors 38 aus, die innerhalb von 125 ms auf eine ansteigende Signalflanke des Meßsignals folgen, die zu einer Schwellwertüberschreitung führt. Der Rauschbegrenzer 40 ist damit flankengetriggert und retriggerbar, das heißt auch innerhalb der Ausblendzeit von 125 ms auftretende Ereignisse lösen die Ausblendzeit erneut aus und verlängern diese damit. Da eine ansteigende Flanke des Meßsignals im Falle der Schwellwertüberschreitung zum Sprung des Ausgangssignals der Schwellwertdetektor von 0 auf 1 führt, wird die Ausblendzeit durch die von 0 auf 1 springende Signalflanke des Ausgangssignals des Schwellwertdetektors 38 ausgelöst. Der Refraktärzeitgeber 42 löst eine nicht retriggerbare Ausblendzeit von 300 ms aus, die mit einer ansteigenden Flanke des Ausgangssignals des Schwellwertdetektors 38 beim Wechsel von logisch "0" auf logisch "1" beginnt. Da diese Ausblendzeit von 300 ms nicht retriggerbar ist, führen Ereignisse innerhalb dieser Refraktärzeit nicht zu einer Verlängerung der Refraktärzeit.

Die zweiten Detektionsmittel 16 umfassen einen Skalierer 44, einen programmierbaren Verstärker 46, eine Absolutwerteinheit 48, einen Schwellwertdetektor 50, einen Rauschbegrenzer 52 und einen Refraktärzeitgeber 54. Auch diese Elemente sind in der Reihenfolge ihres Aufzählens hintereinander geschaltet, so daß der Ausgang eines zunächst genannten Elementes mit dem Eingang des nächst genannten Elementes verbunden ist. Vorzugsweise sind die Elemente der zweiten Detektionsmittel 16 in Form von Software realisiert. Hierzu kann zwischen dem Mittelwertfilter 30 und dem Skalierer 44 ein Ringspeicher oder FIFO-Speicher (FIFO = First In First Out) vorgesehen sein, um einen jeweils aktuellen Signalabschnitt des Ausgangssignals des Mittelwertfilters 30 für die Weiterverarbeitung zwischenzuspeichern. Diese Signalverarbeitung durch die zweiten Detektionsmittel 16 braucht dann nicht in Echtzeit zu erfolgen. Sie erfolgt jedoch streng synchronisiert mit der Signalverarbeitung durch die ersten Detektionsmittel 14.

Der Skalierer 44 skaliert das Ausgangssignal des Mittelwertfilters 30 mit dem Faktor 8. Dementsprechend hat der Skalierer 44 bei einem 11 bit Eingangssignal ein 14 bit Ausgangssignal. Dieses wird durch den programmierbaren Verstärker 46 verstärkt, dessen Verstärkungsfaktor so eingestellt wird, daß er dem anfänglichen Verstärkungsfaktor des programmierbaren Verstärkers 34 der ersten Detektionsmittel 14 entspricht.

Die Absolutwerteinheit 48 bildet aus einem Eingangssignal mit positiven und negativen Signalwerten ein Ausgangssignal mit ausschließlich positiven Signalwerten, deren Betrag dem Betrag der Eingangssignalwerte entspricht. Der Schwellwertdetektor 50 gibt ein Ausgangssignal dann aus, wenn das Eingangssignal einen gespeicherten Schwellwert von 300 überschreitet. Bei Überschreiten des Schwellwertes ändert sich das Ausgangssignal des Schellwertdetektors 50 von logisch "0" auf logisch "1". Die von logisch "0" auf logisch "1" ansteigende Flanke des Schwellwertdetektors 50 veranlaßt den Rauschbegrenzer 52, eine retriggerbare Ausblend- oder Rauschunterdrückungszeitdauer von 50 ms auszulösen. Diese Ausblendzeit ist somit kürzer als die entsprechende Zeit der ersten Detektionsmittel. Sie verlängert sich mit jedem während der Ausblendzeit auftretenden Ereignis. Der Refraktärzeitgeber 54 löst eine nicht retriggerbare Ausblend- oder Refraktärzeit von 135 ms auf jede von 0 auf 1 ansteigende Flanke des Schwellwertdetektors 50 aus, die außerhalb der Refraktärzeit von 135 ms auftritt.

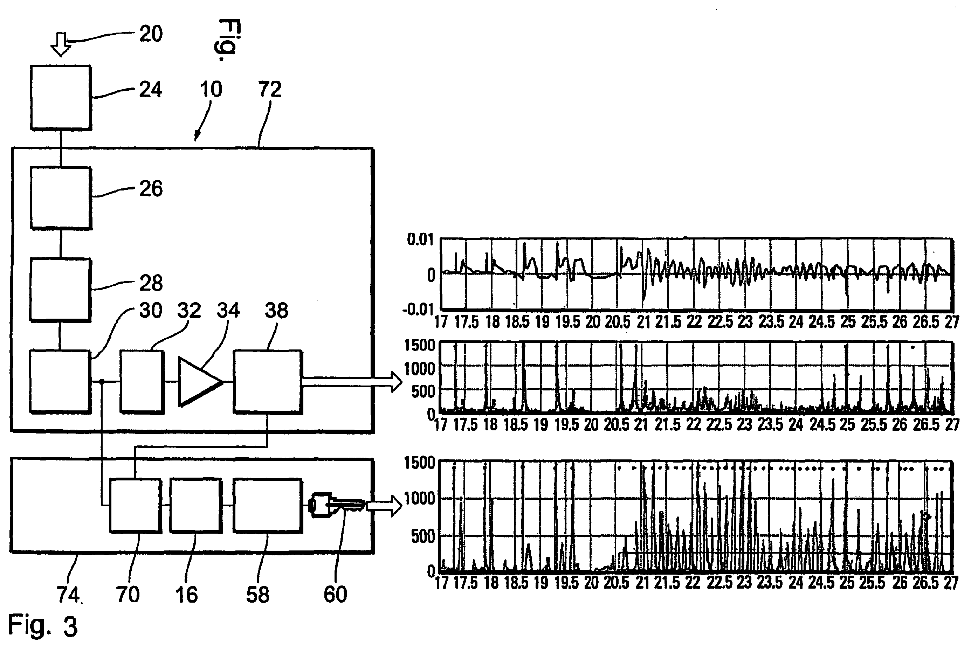

Wie in Figur 3 angedeutet, sind die ersten Detektionsmittel 14 mit den zweiten Detektionsmittel 16 derart verbunden, daß die ersten Detektionsmittel 14 die zweiten Detektionsmittel 16 nur dann starten, wenn die ersten Detektionsmittel eine Herzrate detektieren, die über 140 Schlägen pro Minute liegt. Dieses Auslösen oder Starten der zweiten Detektionsmittel 16 ist retriggerbar. Außerdem werden die zweiten Detekionsmittel 16 durch die ersten Detektionsmittel 14 gestartet, wenn der Verstärkungsfaktor des programmierbaren Verstärkers 34 angepaßt wird. Die zweiten Detektionsmittel 16 werden wieder ausgeschaltet, falls eine Fibrillation detektiert wurde oder falls sie nicht innerhalb von 30 Sekunden wiederausgelöst wurden. Die Anpassung des Verstärkungsfaktors des programmierbaren Verstärkers 34 erfolgt immer dann, wenn der Schwellwertdetektor 38 innerhalb eines vorgegebenen Intervalls keine Ereignisse detektiert und außerdem den Rauschbegrenzer auslösende oder wiederauslösende Ereignisse innerhalb der jeweils letzten 200 ms aufgetreten sind. Der Verstärkungsfaktor wird um einen Schritt herabgesetzt, wenn ein Retriggern, also Wiederauslösen des Rauschbegrenzers 40 durch ein Ereignis innerhalb der Ausblend- oder Rauschunterdrückungszeitdauer auftritt. Zum Unternehmen der in diesem Absatz beschriebenen Funktionen ist eine entsprechend ausgebildete Steuerlogik 56 vorgesehen, die sowohl mit den ersten Detektionsmitteln 14, insbesondere dem programmierbaren Verstärker 34, dem Schwellwertdetektor 38 und dem Rauschbegrenzer 40 sowie den zweiten Detektionsmitteln 16 verbunden.

Die Ausgangssignale des Rauschbegrenzers 40 und des Refraktärzeitgebers 42 der ersten Detektionsmittel 14 sowie die Ausgangssignale des Rauschbegrenzers 52 und des Refraktärzeitgebers 54 der zweiten Detektionsmittel 16 werden der Auswerteeinheit 18 zugeführt. Die Auswerteeinheit 18 umfaßt eine Koinzidenzeinheit 58 und eine Identifikationseinheit 60. Die Koinzidenzeinheit 58 ist mit dem Rauschbegrenzer 40 und dem Refraktärzeitgeber 42 der ersten Detektionsmittel 14 sowie dem Rauschbegrenzer 52 und dem Refraktärzeitgeber 54 der zweiten Detektionsmittel 16 verbunden. Ausgangsseitig gibt die Koinzidenzeinheit 58 ein Signal an die Identifikationseinheit 60 ab. Ausgangssignal der Identifikationseinheit 60 ist das eine Fibrillation kennzeichnende Ausgangssignal der Signalverarbeitungsvorrichtung 10 und deren Ausgang 22.

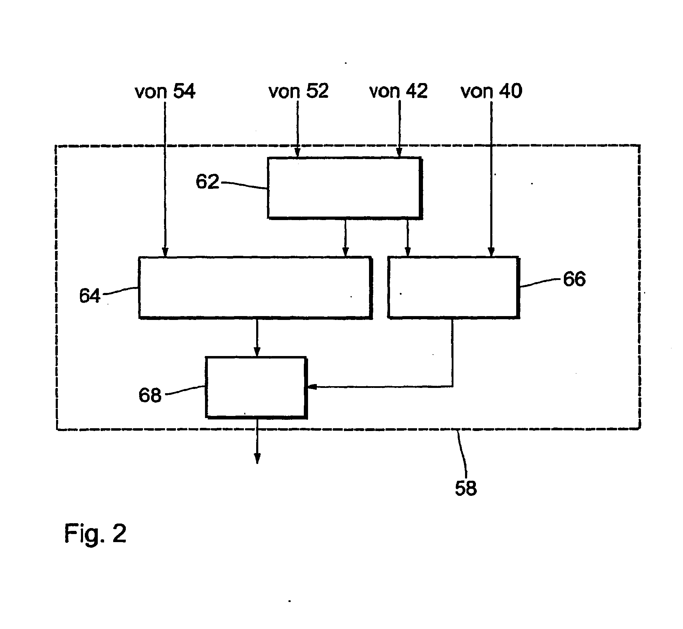

In Figur 2 ist die innere Struktur der Koinzidenzeinheit 58 näher dargestellt. Eine Koinzidenzerkennungseinheit 62 ist mit dem Refraktärzeitgeber 42 der ersten Detektionsmittel 14 und dem Rauschbegrenzer 52 der zweiten Detektionsmittel 16 verbunden. Die Koinzidenzerkennungseinheit 62 ist so ausgebildet, daß in ihrem Ausgang ein Koinzidenzsignal anliegt, wenn ein Ausgangssignal (logisch "1") des Refraktärzeitgebers 52 der zweiten Detektionsmittel 16 innerhalb eines Zeitraumes von 50 ms vor einem Ausgangssignal des Rauschbegrenzers 42 der ersten Detektionsmittel 14 auftritt. Das Ausgangssignal der Koinzidenzerkennungseinheit 62 wird als Koinzidenzsignal sowohl an eine Ausblendeinheit 64 als auch an eine Koinzidenztransfereinheit 66 abgegeben.

Die Ausblendeinheit 64 ist eingangsseitig somit mit der Koinzidenzerkennungseinheit 62 verbunden und außerdem mit dem Refraktärzeitgeber 54 der zweiten Detektionsmittel 16. Die Ausblendeinheit 64 ist so ausgebildet, daß sie all diejenigen Ausgangssignale (logisch "1") der von dem Refraktärzeitgeber 54 ausgegebenen Signalfolge ausblendet, die innerhalb von 300 ms auf ein von der Koinzidenzerkennungseinheit 62 rausgegebenes Koinzidenzsignal folgen. Ausgangssignal der Ausblendeinheit 64 ist somit eine Signalfolge, die ursprünglich von den zweiten Detektionsmitteln 16 stammt und aus der die ausgeblendeten Signalwerte ausgefiltert sind.

Die Koinzidenztransfermittel 66 sind eingangsseitig mit der Koinzidenzerkennungseinheit 62 und dem Rauschbegrenzer 40 der ersten Detektionsmittel 14 verbunden. Die Koinzidenztransfereinheit 66 ist so ausgebildet, daß ein von dem Rauschbegrenzer 40 stammendes Ausgangssignal (logisch "1") dann am Ausgang der Koinzidenztransfermittel 66 anliegt, wenn es innerhalb von 300 ms auf ein von der Koinzidenzerkennungseinheit 62 ausgegebenes Koinzidenzsignal folgt. Genau wie im Ausgang der Ausblendeinheit 64 liegt auch im Ausgang der Ausblendeinheit 66 eine Signalfolge an. Beide Signalfolgen sind miteinander über das Eingangs- oder Meßsignal synchronisiert. Die beiden Signalfolgen liegen am Eingang einer Koinzidenztransferausblendeinheit 68 an, welche so ausgebildet ist, daß die Signale der von der Koinzidenztransfereinheit 66 stammenden Signalfolge der von der Ausblendheit 64 stammenden Signalfolge hinzugefügt werden und alle Signale der von der Ausblendeinheit 64 stammenden Signalfolge ausgeblendet werden, die innerhalb von 300 ms auf ein hinzugefügtes, von der Koinzidenztransfereinheit 66stammendes Signal folgen. Die derart modifizierte Signalfolge liegt im Ausgang der Koinzidenztransferausblendeinheit 68 und gleichzeitig am Ausgang der Koinzidenzeinheit 58 an. Der Ausgang der Koinzidenztransferausblendeinheit 68 und somit der Ausgang der Koinzidenzeinheit 58 sind mit dem Eingang der Identifikationseinheit 60 verbunden.

Die Identifikationseinheit 60 umfaßt Zähler zum Zählen von Intervallen zwischen den Signalen (Markersignalen) der von der Koinzidenztransferausblendeinheit 68 übernommenen Signalfolge. Außerdem umfaßt die Identifikationseinheit einen Intervalldauerbestimmer und einen mit diesem verbundenen Intervallvergleicher. Der Intervallvergleicher hat Zugriff auf einen gespeicherten Wert für eine Vergleichsintervallzeitdauer und markiert alle diejenigen Intervalle der Signalfolge, die kürzer sind als das Vergleichsintervall. Die Identifikationseinheit 60 ist weiterhin so ausgebildet, daß sie mittels der Zähler eine vorgebbare Anzahl von beispielsweise 48 Intervallen abzählt und gleichzeitig die markierten Intervalle innerhalb dieser 48 Intervalle abzählt, die kürzer sind als die Vergleichsintervallzeitdauer. Eine weitere Vergleichseinheit der Identifikationseinheit 60 vergleicht kontinuierlich die Anzahl der markierten Intervalle innerhalb der 48 zuletzt abgezählten Intervalle und vergleicht die Anzahl der markierten Intervalle mit einem gespeicherten Vergleichswert von beispielsweise 36. Ist die Anzahl der markierten Intervalle größer als 36, gibt die Identifikationseinheit 60 über den Signalausgang 22 der Signalverarbeitungsvorrichtung 10 ein Fibrillationserkennungssignal aus. Kriterium für die Ausgabe dieses Fibrillationserkennungssignales ist somit, daß mindestens 36 der 48 jüngsten Intervalle zwischen den Signalen der von der Koinzidenztransferausblendeinheit übernommenen Signalfolge kürzer sind als die Vergleichsintervallzeitdauer.

Zur beispielhaften Erläuterung der Arbeitsweise der Signalverarbeitungsvorrichtung 10 dient Figur 3. Diese zeigt auf der linken Seite eine alternative Darstellung der Signalverarbeitungsvorrichtung 10 aus Figur 1. Die bereits beschriebenen Bestandteile sind mit identischen Bezugszeichen versehen. Zusätzlich dargestellt ist ein Ring- oder FIFO-Speicher 70, der in der Beschreibung zur Figur 1 bereits erwähnt, aber in Figur 1 selbst nicht dargestellt ist. Die Identifikationseinheit 60 ist als Schlüssel dargestellt. Der Block 72 umfaßt die in Form von Hardware realisierten Bestandteile der Signalverarbeitungsvorrichtung 10 und der Block 74 die in Form von Software realisierten Vorrichtungselemente. Bei diesen handelt es sich um Programmodule, die in einem Programmspeicher der Signalverarbeitungsvorrichtung 10 abgespeichert sind und die von einem nicht dargestellten Mikroprozessor verarbeitet werden.

Auf der rechten Seite der Figur 3 ist zu oberst ein Meßsignal dargestellt, wie es am Eingang 20 der Signalverarbeitungsvorrichtung 10 beispielhaft anliegt. Darunter dargestellt ist das durch die Signalaufnahmeeinheit 12 sowie den Hochpassfilter 30, den programmierbaren Verstärker 34 und die Absolutwerteinheit 36 vorverarbeitete Meßsignal, wie es am Eingang des Schwellwertdetektors 38 anliegt. In demselben Diagramm sind am oberen Rand die von dem Schwellwertdetektor 38 ausgegebenen ersten Markersignale dargestellt, die der Schwellwertdetektor 38 immer dann ausgibt, wenn das an seinem Eingang anliegende, im selben Diagramm dargestellte vorverarbeitete Meßsignal den programmierten Schwellwert überschreitet.

Das dritte und zuunterst angeordnete Diagramm auf der rechten Seite der Figur 3 zeigt zum einen das durch die Signalaufnahmeeinheit 12 und die nachfolgenden Elemente der zweiten Detektionsmittel 16 vorverarbeitete Meßsignal, wie es am Eingang des Schwellwertdetektors 50 anliegt. Im oberen Rand des gleichen Diagramms sind die Markersignale eingetragen, die der Schwellwertdetektor 50 immer dann ausgibt, wenn das von den zweiten Detektionsmitteln 16 verarbeitete Meßsignal den programmierten Schwellwert überschreitet. Das zweite und dritte Diagramm auf der rechten Seite von Figur 3 zeigen also neben dem jeweils vorverarbeiteten Meßsignal auch die von dem ersten bzw. dem zweiten Schwellwertdetektor ausgegebene Folgen von ersten und zweiten Markersignalen.

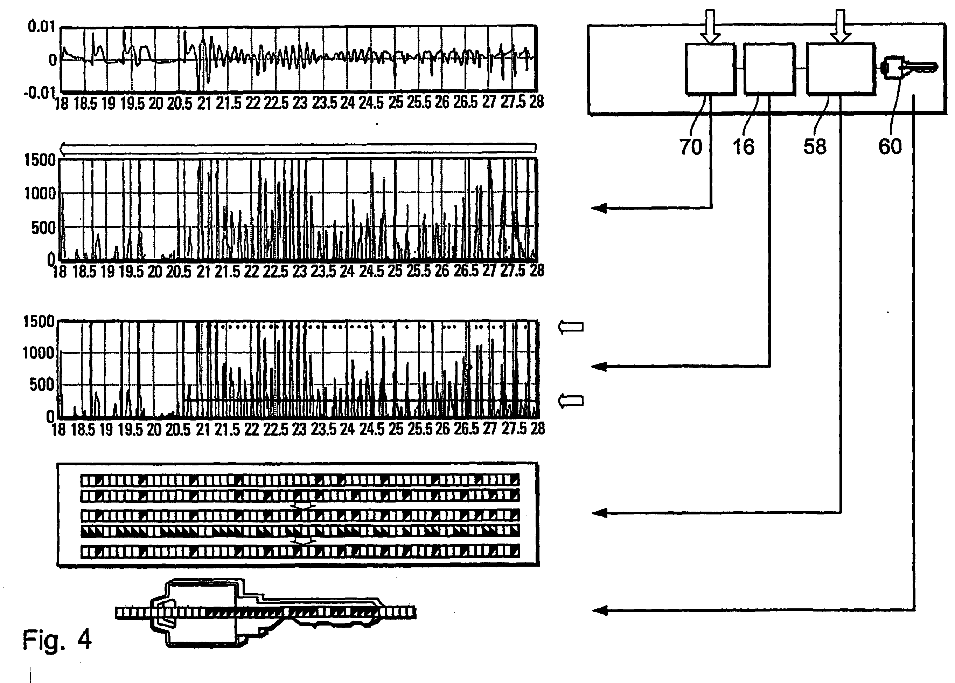

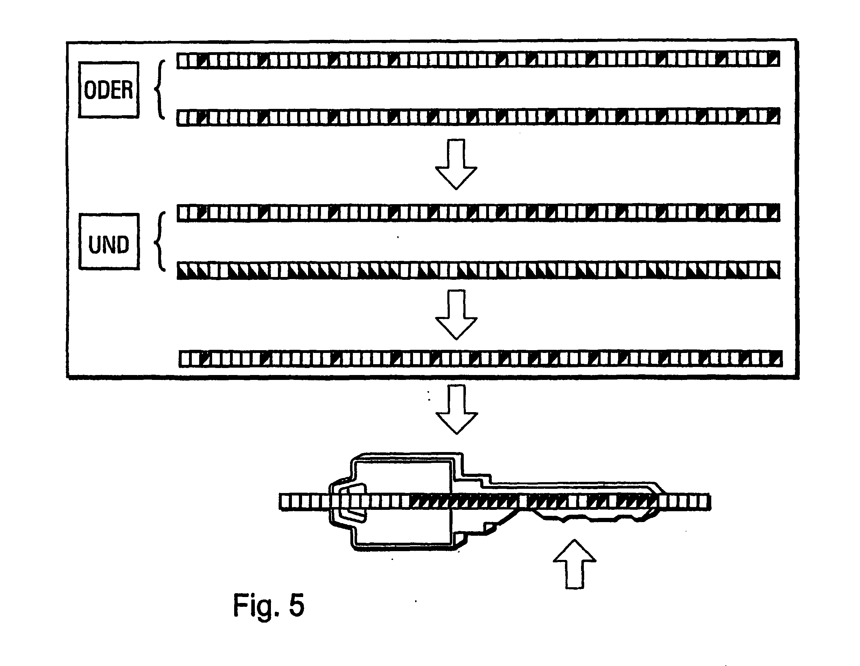

Diese werden durch die Koinzidenzeinheit 58 und die Identifikationseinheit 60 weiterverarbeitet. Dies ist in Figur 4 dargestellt. Figur 4 zeigt auf der linken Seite 4 Diagramme, von denen das oberste das Meßsignal zeigt und dem obersten Diagramm auf der rechten Seite von Figur 3 entspricht. Darunter dargestellt ist der in dem Ringspeicher 70 gespeicherte Signalabschnitt, wie er von der Signalaufnahmeeinheit 12 ausgegeben wird und als Eingangssignal für die zweiten Detektionsmittel 16 dient. Darunter sind das an dem Schwellwertdetektor 50 anliegende, vorverarbeitete Meßsignal und die von dem Schwellwertdetektor 50 ausgegebenen Markersignale dargestellt. Das dritte Diagramm auf der linken Seite von Figur 4 entspricht somit dem dritten Diagramm auf der rechten Seite von Figur 3. Das vierte Diagramm auf der linken Seite von Figur 4 ist eine schematische Darstellung der Folge von Markersignalen, wie sie an verschiedenen Verarbeitungsstufen in der Koinzidenzeinheit 58 vorliegen. Das vierte Diagramm auf der linken Seite von Figur 4 ist in Figur 5 vergrößert dargestellt. Schließlich repräsentiert der in Figur 4 und Figur 5 dargestellte Schlüssel die Signalverarbeitung in der Identifikationseinheit 60.

Von den fünf in Figur 5 dargestellten Signalfolgen ist die erste ein Beispiel für eine Signalfolge, wie sie von den ersten Detektionsmitteln 14 stammt, während die zweite abgebildete Signalfolge einer von den zweiten Detektionsmitteln 16 stammende Signalfolge wie sie am Ausgang der Ausblendeinheit 64 vorliegt, darstellt. Durch die Koinzidenztransfereinheit 66 werden all diejenigen Signale der ersten Signalfolge, die keine Entsprechung in der zweiten Signalfolge haben, detektiert und durch die Koinzidenztransferausblendeinheit 68 in die von der Ausblendeinheit 64 stammende Signalfolge integriert, so daß die dritte in Figur 5 abgebildete Folge entsteht. Die vierte Signalfolge in Figur 5 repräsentiert die zweiten Ausblendzeiten, die durch die Koinzidenztransferausblendeinheit 68 ausgelöst werden. Am Ausgang der Koinzidenztransferausblendeinheit 68 liegt dann ein Signal an, das der fünften in Figur 5 abgebildeten Signalfolge entspricht. Dieses Signal wird von der Identifikationseinheit 60 unter Anwendung des zuvor beschriebenen "36 aus 48"-Kriteriums zur Generierung eines Fibrillationserkennungssignals weiterverarbeitet.

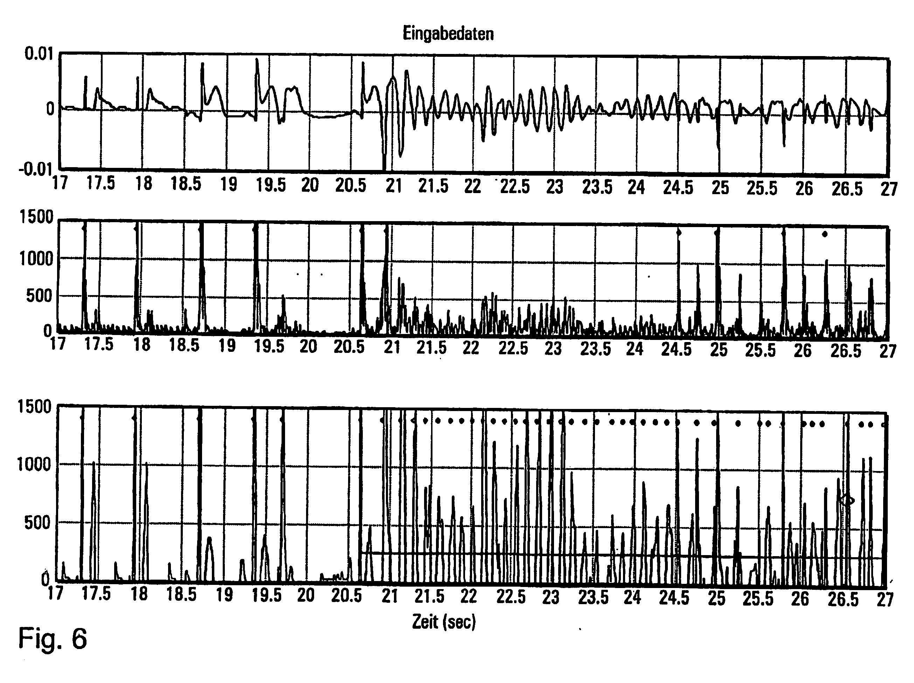

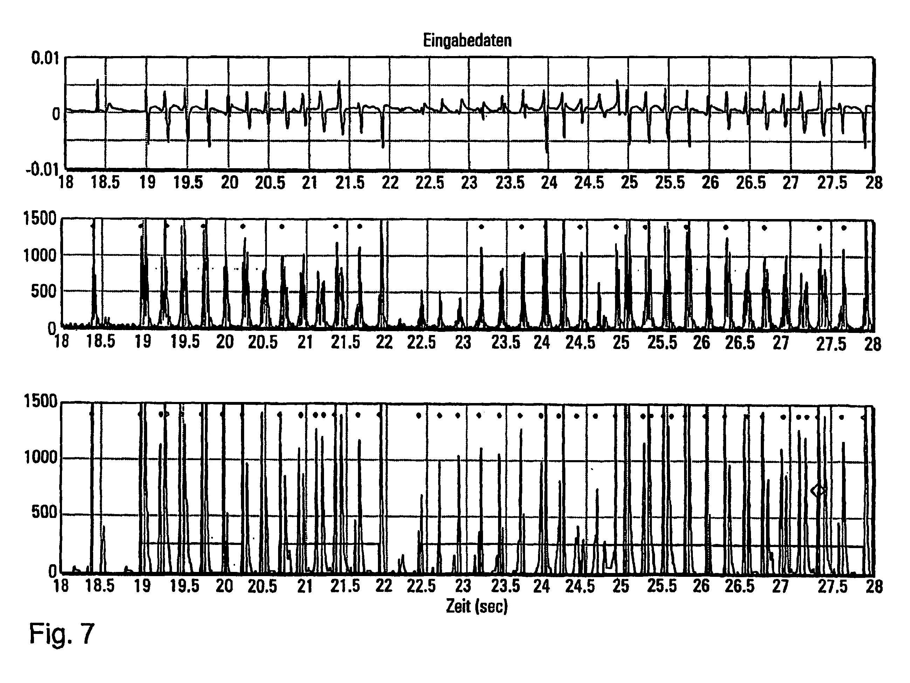

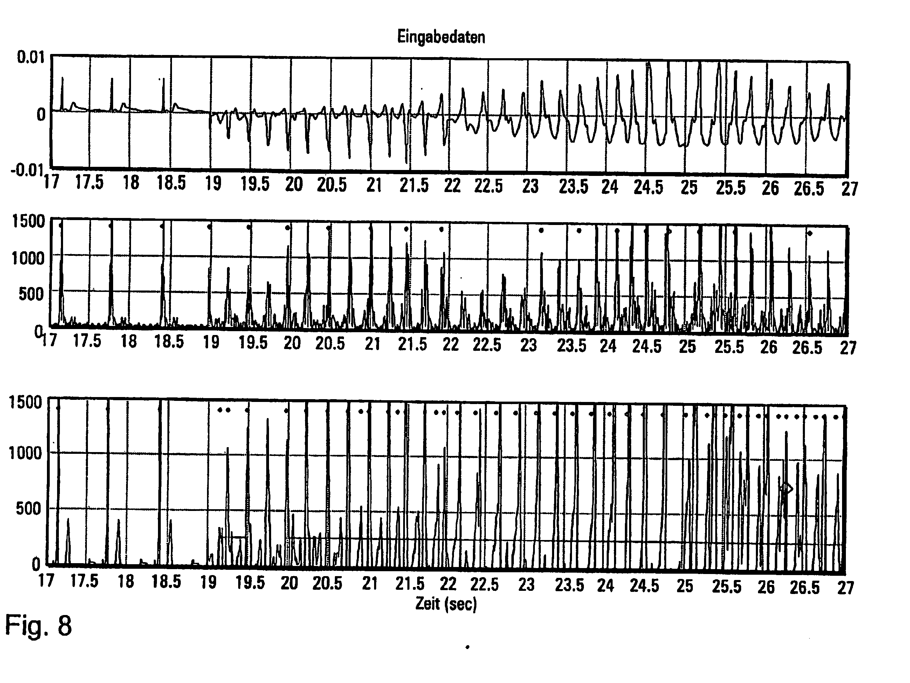

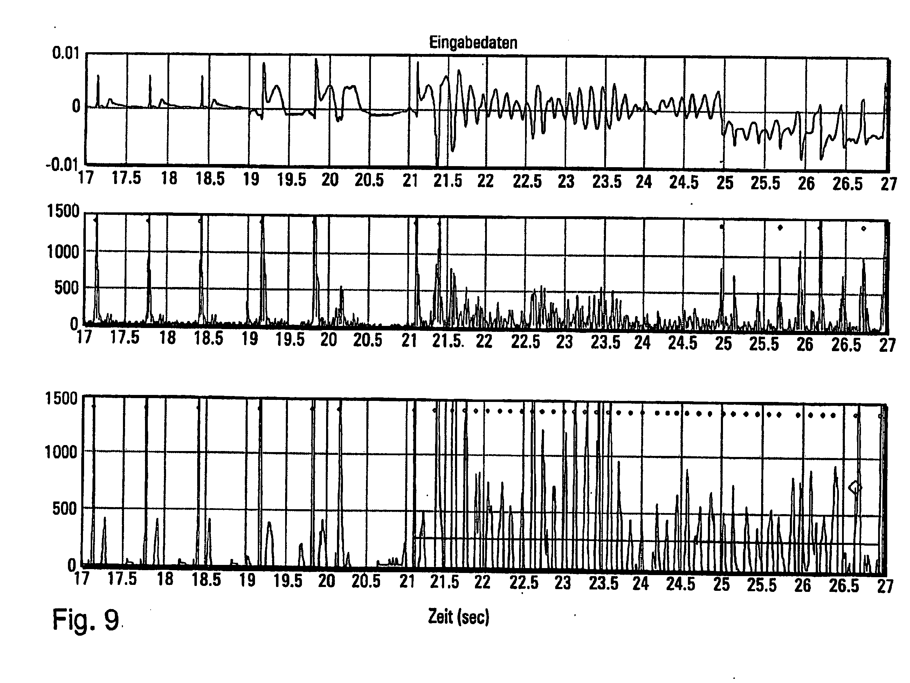

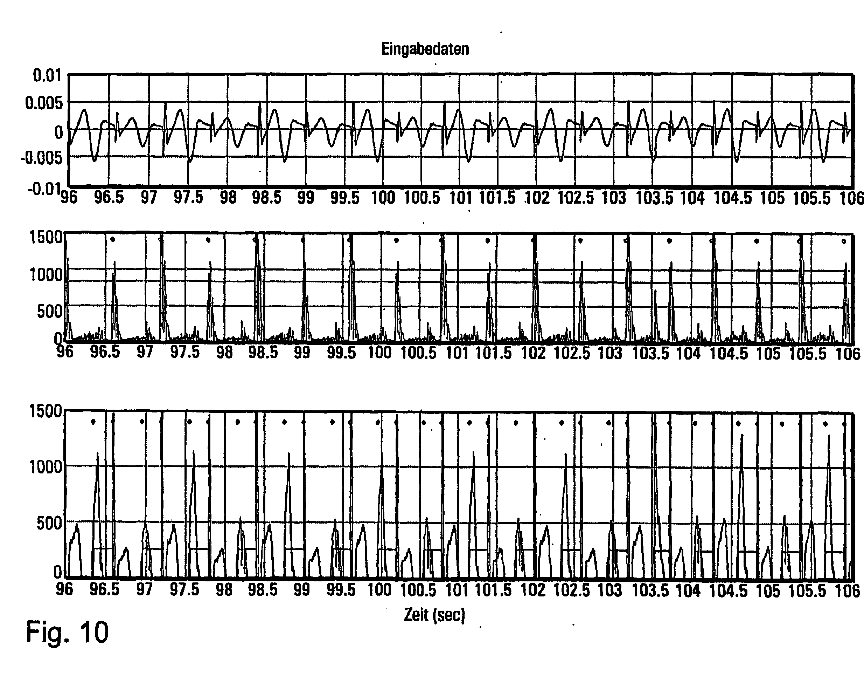

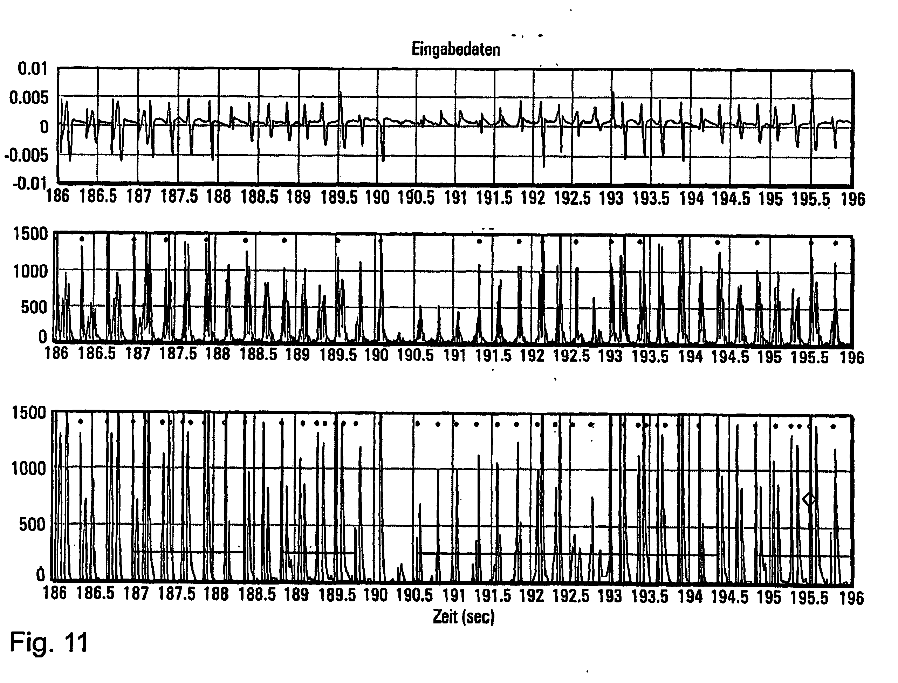

Die in den Figuren 6 bis 11 dargestellten Diagramme entsprechen ihrer Art nach den in Figur 3 auf der rechten Seite dargestellten Diagrammen. Dies bedeutet, daß das erste der jeweils drei Diagramme ein Meßsignal darstellt, das darunter liegende zweite Diagramm das vorverarbeitete Meßsignal am Eingang des Schweilwertdetektors 38 der ersten Detektionsmittel 14 sowie die von dem Schwellwertdetektor 38 ausgegebenen ersten Markersignale. Die ersten Markersignale sind am oberen Rand des jeweils zweiten Diagramms als Rauten dargestellt. Das dritte der jeweils drei Diagramme stellt zum einen das dem im ersten Diagramm dargestellten Meßsignal entsprechende, vorverarbeitete Meßsignal am Eingang des Schwellwertdetektors 50 der zweiten Detektionsmittel 16 dar. Außerdem sind in diesem dritten Diagramm am oberen Rand in rautenförmiger Darstellung die von dem Schwellwertdetektor 50 ausgegebenen zweiten Markersignale dargestellt. Eine abschnittsweise durchgezogene Linie im unteren Drittel des jeweiligen dritten Diagramms kennzeichnet Intervalle, die einer erhöhten Herzrate entsprechen. Eine groß dargestellte Raute in der Mitte des jeweiligen dritten Diagramms repräsentiert das von der Signalverarbeitungsvorrichtung 10 jeweils ausgegebene Fibrillationserkennungssignal.

## Patentansprüche

1. Vorrichtung (10) zur Verarbeitung physiologischer Signale mit
- einer Signalaufnahmeeinheit (12) zur Aufnahme physiologischer Meßsignale, insbesondere zur Aufnahme elektrischer Signale eines Herzens, mit
- ersten Detektionsmitteln (14), die mit der Signalaufnahmeeinheit verbunden sind und Signalbearbeitungsmittel wie einen Filter (32) sowie einen ersten Schwellwertspeicher sowie eine mit diesem verbundene Vergleichseinheit (38) umfassen, die zum Ausgeben eines ersten Markersignals ausgebildet ist, falls die Amplitude des Meßsignals einen im ersten Schwellwertspeicher gespeicherten Schwellwert überschreitet, mit
- zweiten Detektionsmitteln (16), die mit der Signalaufnahmeeinheit verbunden sind, und einen zweiten Schwellwertspeicher sowie eine mit diesem verbundene zweite Vergleichseinheit (50) umfassen, die zum Ausgeben eines zweiten Markersignals ausgebildet ist, falls die Amplitude des Meßsignals einen im zweiten Schwellwertspeicher gespeicherten Schwellwert überschreitet, sowie mit
- einer Auswerteeinheit (18), die mit den ersten und den zweiten Detektionsmitteln zur Übernahme jeweils einer Folge der ersten und der zweiten Markersignale verbunden und zur Verarbeitung der Folgen von Markersignalen zu einer einzigen gefilterten Folge von Markersignalen, zur Analyse der zeitlichen Abfolge der Markersignale, insbesondere der Intervalle zwischen Markersignalen der gefilterten Folge und Ausgabe eines von der Analyse abhängigen Erkennungssignals ausgebildet ist.

2. Vorrichtung nach Anspruch 1, **dadurch gekennzeichnet, daß** die Auswerteeinheit (18, 60) einen Zähler umfaßt, der zum Zählen der Markersignale oder der Intervalle zwischen Markersignalen innerhalb eines vorgegebenen Abschnitts der gefilterten Folge von Markersignalen ausgebildet ist.

3. Vorrichtung nach Anspruch 1 oder 2, **dadurch gekennzeichnet, daß** die ersten Detektionsmittel (14) eine erste Absolutwerteinheit (36) umfaßt, die zum Bilden des Absolutwertes des Meßsignals ausgebildet ist.

4. Vorrichtung nach Anspruch 3, **dadurch gekennzeichnet, daß** die erste Absolutwerteinheit (36) der ersten Vergleichseinheit (38) vorgeschaltet ist.

5. Vorrichtung nach einem der Ansprüche 1 bis 4, **dadurch gekennzeichnet, daß** die zweiten Detektionsmittel (16) eine zweite Absolutwerteinheit (48) umfaßt, die zum Bilden des Absolutwertes des Meßsignals ausgebildet ist.

6. Vorrichtung nach Anspruch 5, **dadurch gekennzeichnet, daß** die zweite Absolutwerteinheit (48) derzweiten Vergleichseinheit (50) vorgeschaltet ist.

7. Vorrichtung nach einem der Ansprüche 1 bis 6, **dadurch gekennzeichnet, daß** die Signalaufnahmeeinheit (12) einen Hochpassfilter (24) mit einer bevorzugten Grenzfrequenz in der Größenordnung von 10Hz umfaßt.

8. Vorrichtung nach einem der Ansprüche 1 bis 6, **dadurch gekennzeichnet, daß** die zweiten Detektionsmittel (16) einen Hochpassfilter mit einer bevorzugten Grenzfrequenz in der Größenordnung von 10Hz umfaßen.

9. Vorrichtung nach Anspruch 7 oder 8, **dadurch gekennzeichnet, daß** die Signalbearbeitungsmittel der ersten Detektionsmittel (14) einen Hochpaßfilter (32) mit einer Grenzfrequenz aufweisen, die über derjeneigen des Hochpassfilters (24) der Signalaufnahmeeinheit (12) oder der zweiten Detektionsmittel (16) liegt und vorzugsweise die Größenordnung von 20 bis 30 Hz hat.

10. Vorrichtung nach einem der Ansprüche 1 bis 9, **dadurch gekennzeichnet, daß** die ersten Detektionsmittel (14) eine erste Rauschbegrenzungseinheit (40) umfassen, die ausgebildet ist, die Weiterverarbeitung des Meßsignals innerhalb einer vorgebbaren ersten Rauschunterdrückungszeitdauer in der Größenordnung von vorzugsweise 125 ms nach jedem Signalwert des Meßsignals zu unterdrücken, **daß** zu einem ersten Markersignal führt. (damit retriggerbar)

11. Vorrichtung nach einem der Ansprüche 1 bis 10, **dadurch gekennzeichnet, daß** die zweiten Detektionsmittel (16) eine zweite Rauschbegrenzungseinheit (52) umfassen, die ausgebildet ist, die Weiterverarbeitung des Meßsignals innerhalb einer vorgebbaren zweiten Rauschunterdrückungszeitdauer von vorzugsweise 50 ms nach jedem Signalwert des Meßsignals zu unterdrücken, **daß** zu einem zweiten Markersignal führt.

12. Vorrichtung nach einem der Ansprüche 1 bis 11, **dadurch gekennzeichnet, daß** die ersten Detektionsmittel (14) einen ersten Refraktärzeitgeber (42) für eine erste Refraktärzeit umfassen, der ausgebildet ist, nach jedem außerhalb der ersten Refraktärzeit liegendem Signalwert, der zu einem ersten Markersignal führt, eine vorgebbare absolute Refraktärzeit auszulösen, die vorzugsweise eine Größenordnung von 150 ms hat, und **daß** die ersten Detektionsmittel (14) ausgebildet sind, die Weiterverarbeitung des Meßsignales innerhalb der absoluten Refraktärzeit zu unterdrücken.

13. Vorrichtung nach Anspruch 12, **dadurch gekennzeichnet, daß** der erste Refraktärzeitgeber (42) ausgebildet ist, eine gesamte Refraktärzeit aufzulösen, die größer ist als die absolute Refraktärzeit, diese einschließt und vorzugsweise 300 ms beträgt, und **daß** die ersten Detektionsmittel (14) ausgebildet sind, die Weiterverarbeitung von ersten Markersignalen innerhalb der Refraktärzeit zu unterdrücken.

14. Vorrichtung nach Anspruch 12 oder 13, **dadurch gekennzeichnet, daß** die zweiten Detektionsmittel (16) einen zweiten Refraktärzeitgeber (52) für eine zweite Refraktärzeit umfassen, der ausgebildet ist, nach jedem außerhalb der zweiten Refraktärzeit liegendem Signalwert, der zu einem zweiten Markersignal führt, eine vorgebbare Refraktärzeit auszulösen, die kleiner oder gleicher der Refraktärzeit der ersten Detektionmittel (14) ist, und **daß** die zweiten Detektionsmittel (16) ausgebildet sind, die Weiterverarbeitung von zweiten Markersignalen innerhalb der Refraktärzeit zu unterdrücken.

15. Vorrichtung nach einem der Ansprüche 10 bis 14, **gekennzeichnet durch** Flankendetektionsmittel, die mit der ersten und/oder derzweiten Rauschunterdrückungseinheit (40, 52) und/oder dem ersten und/oder dem zweiten Refraktärzeitgeber (42, 54)verbunden und ausgebildet sind, eine ansteigende Signalflanke des Meßsignals zu detektieren, der einem Meßsignalwert zugeordnet ist, welcher einem ersten oder zweiten Markersinal zugeordnet ist, derart, daß die jeweilige Rauschunterdrückungszeitdauer und oder die jeweilge Refraktärzeit mit der detektierten Signalflanke beginnt.

16. Vorrichtung nach Anspruch 15, **dadurch gekennzeichnet, daß** die Flankendetektionsmittel mit dem ersten und/oder dem zweiten Schwellwertspeicher verbunden und ausgebildet sind, eine positive Signalflanke durch einen aufsteigenden Schwellwertdurchgang des Meßsignals zu detektieren.

17. Vorrichtung nach einem der Ansprüche 1 bis 16, **dadurch gekennzeichnet, daß** die Auswerteeinheit (18) zur zeitlich korrelierten Übernahme von Folgen erster und zweiter Markersignale ausgebildet ist und eine Logikeinheit (58) umfaßt, die ausgebildet ist, aus beiden Folgen eine einzige Ausgangsfolge der Markersignale zu bilden, die die sowohl alle ersten als auch alle zweiten Markersignale entsprechend ihrer Zuordnung zu den auslösenden Meßsignalwerten zeitlich geordnet enthält, wobei erste und zweite Meßsignale innerhalb einer vorgebbarn Koinzidenzzeit von vorzugsweise 50 ms vor und/oder nach einem ersten Markersignal zu einem Markersignal der Ausgangsfolge zusammengefaßt sind.

18. Vorrichtung nach Anspruch 17, **dadurch gekennzeichnet, daß** die Auswerteeinheit (18) eine Filtereinheit umfaßt, die mit der Logikeinheit verbunden und ausgebildet ist, eine gefilterte Folge von Markersignalen derart zu bilden, **daß** die zusammengesetzte Folge nur solche Markersignale enthält, die außerhalb einer Ausblendzeit von vorzugsweise 300 ms liegen, die mit jedem Markersignal aus der Folge von zweiten Markersignalen beginnt.

19. Vorrichtung nach einem der Ansprüche 1 bis 16, **dadurch gekennzeichnet, daß** die Auswerteeinheit (18) zur zeitlich korrelierten Übernahme von Folgen erster und zweiter Markersignale ausgebildet ist und
- Koinzidenzerkennungsmittel (62) umfaßt, die ausgebildet sind, ein Koinzidenzsignal auszugeben, wenn eines der ersten Markersignale außerhalb der ersten Refraktärzeit und eines der zweiten Markersignale außerhalb der zweiten Rauschunterdrückungszeitdauer in einen Koinzidenzzeitraum von vorzugsweise 50 ms fallen, sowie
- Ausblendmittel (64), die mit den Koinzidenzerkennungsmitteln verbunden und ausgebildet sind, solche der zeiten Markersignale für die weitere Signalverarbeitung auszublenden, die innerhalb einer ersten Ausblendzeit von vorzugsweise 300 ms auf ein zweites Markersignal folgen, welches zu einem Koinzidenzsignal geführt hat, sowie
- Koinzidenztransfermittel (66), die mit den Koinzidenzerkennungsmitteln (62) verbunden und ausgebildet sind, das erste der ersten Markersignale, welches außerhalb der ersten Rauschunterdrückungszeitdauer und innerhalb einer Transferzeitdauer von vorzugsweise 300 ms auf ein Koinzidenzsignal folgt, unter Beibehalt des Zeitzusammenhanges der Folge der zweiten Markersignale hinzuzufügen und so eine modifizierte Folge von Markersignalen zu bilden,
- Koinzidenztransferausblendmittel (68), die mit den Koinzidenztransfermitteln (66) verbunden und ausgebildet sind, aus der modifizierten Folge von Markersignalen all diejenigen Markersignale auszublenden, die innerhalb einer zweiten Ausblendzeit von vorzugsweise 300 ms auf ein von den Koinzidenztransfermitteln hinzugefügtes Markersignal folgen, um so eine zusammengesetzte Folge von Markersignalen zu bilden.

20. Vorrichtung nach Anspruch 18 oder 19, **dadurch gekennzeichnet, daß** die Auswerteeinheit (18) eine Identifikationseinheit (60) umfaßt, die
- Intervallbestimmungsmittel umfaßt, welche ausgebildet sind, die Intervalldauer zwischen zwei benachbarten Markersignalen der gefilterten Folge von Markersignalen liegender Zeitintervalle zu bestimmen, sowie einen
- Vergleichsintervallspeicher für eine Vergleichsintervallzeitdauer und
- Intervallvergleichsmittel, die mit den Intervallbestimmungsmitteln sowie dem Vergleichsintervallspeicher verbunden und ausgebildet sind, diejenigen Intervalle der gefilterten Folge von Markersignalen zu detektieren, die kürzer sind, als die Vergleichsintervallzeitdauer,
- einem Zähler, der mit den Intervallvergleichsmitteln verbunden und ausgebildet ist, die innerhalb eines Abschnitts der gefilterten Folge von Markersignalen liegende Anzahl von Markersignalen oder Intervallen zwischen jeweils zwei benachbarten Markersignalen zu bestimmen sowie die Anzahl dieser Intervalle, die kürzer sind, als die Vergleichsintervallzeitdauer, und
- einen Signalgeber, der mit der Intervallzähleinheit und einem Quotientenspeicher verbunden und derart ausgebildet ist, **daß** der Signalgeber ein Signal ausgibt, falls der Anteil der Intervalle, die kürzer als die Vergleichsintervallzeitdauer sind, einen in dem Quotientenspeicher gespeicherten Anteil an der Gesamtzahl von Intervallen eines Abschnittes der gefilterten Folge überschreitet.

21. Vorrichtung nach einem der Ansprüche 1 bis 20, **dadurch gekennzeichnet, daß** die Signalaufnahmeeinheit (12) einen Dezimator (26) aufweisen, der das Meßsignal vorzugsweise in einem 4096Hz-Takt abtastet und vorzugsweise in einem 512Hz-Takt einen jeweils neuen Abtastwert für das Meßsignal ausgibt.

22. Vorrichtung nach einem der Ansprüche 1 bis 21, **dadurch gekennzeichnet, daß** die Signalaufnahmeeinheit (12) einen Analog/Digital-Wandler (28) umfaßt, der ausgebildet ist, jeden analogen Abtastwert, insbesondere die Ausgangswerte des Dezimators (26), in einen Digitalwert zu wandeln, so **daß** am Ausgang des Digital/Analogwandlers eine zeitdiskrete Folge digitaler Werte anliegt, die dem Meßsignal entsprechen.

23. Vorrichtung nach einem der Ansprüche 1 bis 22, **dadurch gekennzeichnet, daß** die Signalaufnahmeeinheit (12) Mittelwertbildungsmittel (30) zur Bildung eines gleitenden Mittelwertes des Meßsignals umfaßt.

24. Vorrichtung nach einem der Ansprüche 1 bis 23, **dadurch gekennzeichnet, daß** die ersten Detektionsmittel (14) einen Signalverstärker (32) mit anpaßbarem Verstärkungsfaktor umfaßt.

25. Vorrichtung nach Anspruch 24, mit einer Stimulationseinheit zur Abgabe von zur Herzstimulation geeigneten Impulsen, **dadurch gekennzeichnet, daß** der Signalverstärker ausgebildet ist, den Verstärkungsfaktor zu erhöhen, falls die Stimulationseinheit einen Impuls abgibt und/oder die Folge zweiter Markersignale ein Markersignal innerhalb eines vorgegebenen Zeitraums von vorzugsweise 200 ms vor Abgabe eines Impulses aufweist.

26. Vorrichtung nach Anspruch 25, **gekennzeichnet durch** eine Aktivierungseinheit, die mit den zweiten Detektionsmitteln sowie dem Signalverstärker derart verbunden ist, daß sie die zweiten Detektionsmittel aktiviert, falls der Signalverstärker den Verstärkungsfaktor erhöht.

27. Vorrichtung nach einem der Ansprüche 1 bis 25, **gekennzeichnet durch** eine Aktivierungseinheit (56), die mit den ersten und den zweiten Detektionsmitteln derart verbunden ist, daß sie die zweiten Detektionsmittel aktiviert, wenn die Folge der ersten Markersignale eine vorgebene Frequenz von vorzugsweise 140 Markersignalen pro Minute überschreitet.

28. Vorrichtung nach einem der Ansprüche 1 bis 27, **dadurch gekennzeichnet, daß** die zweiten Detektionsmittel einen Signalspeicher umfassen, der über einen Analog-Digitalwandler mit der Signalaufnahmeeinheit verbunden und zum Speichern zumindest eines jeweils aktuellen Abschnittes des Meßsignals ausgebildet ist.

## Claims

1. A device (10) for processing physiological signals having
- a signal recording unit (12) for recording physiological measured signals, particularly for recording electrical signals of a heart, having
- first detection means (14), which are connected to the signal recording unit and comprise signal processing means such as a filter (32) as well as a first threshold value memory and a comparison unit (38) connected thereto, which is implemented to output a first marker signal if the amplitude of the measured signal exceeds a threshold value stored in the first threshold value memory, having
- second detection means (16), which are connected to the signal recording unit, and comprise a second threshold value memory and a second comparison unit (50) connected thereto, which is implemented to output a second marker signal if the amplitude of the measured signal exceeds a threshold value stored in the second threshold value memory, and having
- an analysis unit (18) which is connected to the first and the second detection means to receive a sequence of each of the first and the second marker signals, respectively, and is implemented to process the sequences of marker signals to produce a single filtered sequence of marker signals, to analyze the chronological sequence of the marker signals, in particular the intervals between marker signals of the filtered sequence, and to output a recognition signal as a function of the analysis.

2. The device according to Claim 1, **characterized in that** the analysis unit (18, 60) comprises a counter, which is implemented to count the marker signals or the intervals between marker signals within a predefined section of the filtered sequence of marker signals.

3. The device according to Claim 1 or 2, **characterized in that** the first detection means (14) comprise a first absolute value unit (36), which is implemented to produce the absolute value of the measured signal.

4. The device according to Claim 3, **characterized in that** the first absolute value unit (36) is connected upstream from the first comparison unit (38).

5. The device according to one of Claims 1 through 4, **characterized in that** the second detection means (16) comprise a second absolute value unit (48), which is implemented to produce the absolute value of the measured signal.

6. The device according to Claim 5, **characterized in that** the second absolute value unit (48) is connected upstream from the second comparison unit (50).

7. The device according to one of Claims 1 through 6, **characterized in that** the signal recording unit (12) comprises a high-pass filter (24) having a preferred limiting frequency in the order of magnitude of 10 Hz.

8. The device according to one of Claims 1 through 6, **characterized in that** the second detection means (16) comprise a high-pass filter having a preferred limiting frequency in the order of magnitude of 10 Hz.

9. The device according to Claim 7 or 8, **characterized in that** the signal processing means of the first detection means (14) have a high-pass filter (32) having a limiting frequency which is above that of the high-pass filter (24) of the signal recording unit (12) or the second detection means (16) and preferably has an order of magnitude of 20 to 30 Hz.

10. The device according to one of Claims 1 through 9, **characterized in that** the first detection means (14) comprise a first noise limiting unit (40), which is implemented to suppress the further processing of the measured signal within a predefinable first noise suppression period, preferably in the order of magnitude of 125 ms, after every signal value of the measured signal which results in a first marker signal (thus may be retriggered).

11. The device according to one of Claims 1 through 10, **characterized in that** the second detection means (16) comprise a second noise limiting unit (52), which is implemented to suppress the further processing of the measured signal within a predefinable second noise suppression period, preferably of 50 ms, after every signal value of the measured signal which results in a second marker signal.

12. The device according to one of Claims 1 through 11, **characterized in that** the first detection means (14) comprise a first refractory timer (42) for a first refractory time, which is implemented to trigger a predefinable absolute refractory time, which preferably has an order of magnitude of 150 ms, after every signal value, lying outside the first refractory time, which results in a first marker signal, and the first detection means (14) are implemented to suppress the further processing of the measured signal within the absolute refractory time.

13. The device according to Claim 12, **characterized in that** the first refractory timer (42) is implemented to trigger an overall refractory time which is longer than the absolute refractory time, includes it, and is preferably 300 ms, and the first detection means (14) are implemented to suppress the further processing of first marker signals within the refractory time.

14. The device according to Claim 12 or 13, **characterized in that** the second detection means (16) comprise a second refractory timer (52) for a second refractory time, which is implemented to trigger a predefinable refractory time, which is less than or equal to the refractory time of the first detection means (14), after every signal value which results in a second marker signal and lies outside the second refractory time, and the second detection means (16) are implemented to suppress the further processing of second marker signals within the refractory time.

15. The device according to one of Claims 10 through 14, **characterized by** flank detection means, which are connected to the first and/or the second noise suppression unit (40, 52) and/or the first and/or the second refractory timer (42, 54) and are implemented to detect a rising signal flank of the measured signal which is assigned to a measured signal value which is assigned to a first or second marker signal in such a way that the particular noise suppression period and/or the particular refractory time begins with the detected signal flank.

16. The device according to Claim 15, **characterized in that** the flank detection means are connected to the first and/or the second threshold value memory and are implemented to detect a positive signal flank through a rising threshold value passage of the measured signal.

17. The device according to one of Claims 1 through 16, **characterized in that** the analysis unit (18) is implemented for chronologically correlated reception of sequences of first and second marker signals and comprises a logic unit (58), which is implemented to form a single output sequence of the marker signals from both sequences, which contains both all first and also all second marker signals chronologically ordered in accordance with their assignment to the triggering measured signal values, first and second measured signals within a predefinable coincidence time, preferably of 50 ms, before and/or after a first marker signal, being summarized into one marker signal of the output sequence.

18. The device according to Claim 17, **characterized in that** the analysis unit (18) comprises a filter unit which is connected to the logic unit and is implemented to form a filtered sequence of marker signals in such a way that the summarized sequence only contains those marker signals which are lying outside a skipping time, preferably of 300 ms, which begins with every marker signal from the sequence of second marker signals.

19. The device according to one of Claims 1 through 16, **characterized in that** the analysis unit (18) is implemented for chronologically correlated reception of sequences of first and second marker signals and
- comprises coincidence recognition means (62), which are implemented to output a coincidence signal if one of the first marker signals falls outside the first refractory time and one of the second marker signals falls outside the second noise suppression period in a coincidence time span, preferably of 50 ms, and
- skipping means (64), which are connected to the coincidence recognition means and are implemented to skip for further signal processing those of the second marker signals which follow within a first skipping time, preferably of 300 ms, on a second marker signal which has resulted in a coincidence signal, and
- coincidence transfer means (66), which are connected to the coincidence recognition means (62) and are implemented to add the first of the first marker signals which follow a coincidence signal outside the first noise suppression period and inside a transfer duration, preferably of 300 ms, while maintaining the time relationship of the sequence of second marker signals and thus form a modified sequence of marker signals,
- coincidence transfer skipping means (68), which are connected to the coincidence transfer means (66) and are implemented to skip all those marker signals from the modified sequence of marker signals which follow a marker signal added by the coincidence transfer means within a second skipping time, preferably of 300 ms, in order to thus form a summarized sequence of marker signals.

20. The device according to Claim 18 or 19, **characterized in that** the analysis unit (18) comprises an identification unit (60), which
- comprises interval determination means, which are implemented to determine the interval period of time intervals lying between two neighboring marker signals of the filtered sequence of marker signals, and a
- comparison interval memory for a comparison interval period, and
- interval comparison means, which are connected to the interval determination means and the comparison interval memory and are implemented to detect those intervals of the filtered sequence of marker signals which are shorter than the comparison interval period,
- a counter which is connected to the interval comparison means and is implemented to determine the number of marker signals lying within a section of the filtered sequence of marker signals or intervals between each two neighboring marker signals as well as the number of these intervals which are shorter than the comparison interval period, and
- a signal transmitter, which is connected to the interval counter unit and a quotient memory and is implemented in such a way that the signal transmitter outputs a signal if the component of the intervals which are shorter than the comparison interval period exceeds a component of the total number of intervals of a section of the filtered sequence stored in the quotient memory.

21. The device according to one of Claims 1 through 20, **characterized in that** the signal recording unit (12) has a decimator (26), which samples the measured signal, preferably in a 4096 Hz cycle, and preferably outputs a new sampling value for the measured signal in a 512 Hz cycle.

22. The device according to one of Claims 1 through 21, **characterized in that** the signal recording unit (12) comprises an analog/digital converter (28) which is implemented to convert every analog sampling value, in particular the output values of the decimator (26), into a digital value, so that a time-discrete sequence of digital values, which correspond to the measured signal, is applied to the output of the digital/analog converter.

23. The device according to one of Claims 1 through 22, **characterized in that** the signal recording unit (12) comprises mean value calculation means (30) for calculating a sliding mean value of the measured signal.

24. The device according to one of Claims 1 through 23, **characterized in that** the first detection means (14) comprises a signal amplifier (32) having adaptable amplification factor.

25. The device according to Claim 24, having a stimulation unit for delivering pulses suitable for cardiac stimulation, **characterized in that** the signal amplifier is implemented to increase the amplification factor if the stimulation unit delivers a pulse and/or the sequence of second marker signals has a marker signal within a predefinable time span, preferably of 200 ms, before delivery of a pulse.

26. The device according to Claim 25, **characterized by** an activation unit which is connected to the second detection means and the signal amplifier in such way that it activates the second detection means if the signal amplifier increases the amplification factor.

27. The device according to one of Claims 1 through 25, **characterized by** an activation unit (56), which is connected to the first and the second detection means in such way that it activates the second detection means if the sequence of the first marker signals exceeds a predefined frequency, preferably of 140 marker signals per minute.

28. The device according to one of Claims 1 through 27, **characterized in that** the second detection means comprise a signal memory which is connected to the signal recording unit via an analog/digital converter and is implemented to store at least one current section of the measured signal at a time.

## Revendications

1. Un dispositif (10) pour le traitement de signaux physiologiques comprenant
- une unité d'enregistrement de signal (12) pour l'enregistrement de signaux de mesure physiologiques, en particulier pour l'enregistrement de signaux électriques d'un coeur, avec
- des premiers moyens de détection (14), qui sont reliés à l'unité d'enregistrement de signal et comprennent des moyens de traitement de signal comme un filtre (32) ainsi qu'une première mémoire de valeur seuil et une unité de comparaison (38) reliée à cette mémoire, qui est conçue pour l'édition d'un premier signal de marqueur, dans le cas où l'amplitude du signal de mesure dépasse une valeur seuil mémorisée dans la première mémoire de la valeur seuil, avec
- des seconds moyens de détections (16), qui sont reliés à l'unité d'enregistrement de signal, et comprennent une seconde mémoire de valeur seuil ainsi qu'une seconde unité de comparaison (50) reliée à cette mémoire, qui est conçue pour l'édition d'un second signal de marqueur dans le cas où l'amplitude du signal de mesure dépasse une valeur seuil mémorisée dans la seconde mémoire de valeur seuil, ainsi qu'avec
- une unité d'analyse (18), qui est reliée aux premiers et aux seconds moyens de détection pour la prise en charge de respectivement une séquence des premiers et des seconds signaux de marqueur et est conçue pour le traitement de séquences des signaux de marqueur pour former une seule fréquence filtrée de signaux de marqueur, pour l'analyse de la séquence dans le temps des signaux de marqueur, en particulier des intervalles entre des signaux de marqueur de la fréquence filtrée et l'édition d'un signai de reconnaissance dépendant de l'analyse.

2. Dispositif selon la revendication 1, **caractérisé en ce que** l'unité d'analyse (18, 60) comprend un compteur, qui est conçu pour le comptage des signaux de marqueur et des intervalles entre des signaux de marqueur à l'intérieur d'une période prédéfinie de la séquence filtrée de signaux de marqueur.

3. Dispositif selon la revendication 1 ou 2, **caractérisé en ce que** les premiers moyens de détection (14) comprennent une première unité de valeur absolue (36), qui est conçue pour la formation de la valeur absolue du signal de mesure.

4. Dispositif selon la revendication 3, **caractérisé en ce que** la première unité de valeur absolue (36) est montée en amont de la première unité de comparaison (38).

5. Dispositif selon l'une quelconque des revendications 1 à 4, **caractérisé en ce que** les seconds moyens de détection (16) comprennent une seconde unité de valeur absolue (48), qui est conçue pour la formation de la valeur absolue du signal de mesure.

6. Dispositif selon la revendication 5, **caractérisé en ce que** la seconde unité de valeur absolue (48) est montée en amont de la seconde unité de comparaison (50).

7. Dispositif selon l'une quelconque des revendications 1 à 6, **caractérisé en ce que** l'unité d'enregistrement de signal (12) comprend un filtre passe-haut (24) avec une fréquence limite préférée de l'ordre de 10 Hz.

8. Dispositif selon l'une quelconque des revendications 1 à 6, **caractérisé en ce que** les seconds moyens de détection (16) comprennent un filtre passe-haut avec une fréquence limite préférée de l'ordre de 10 Hz.

9. Dispositif selon la revendication 7 ou 8, **caractérisé en ce que** les moyens de traitement de signal des premiers moyens de détection (14) présentent un filtre passe-haut (32) avec une fréquence limite, qui est supérieure à celle du filtre passe-haut (24) de l'unité d'enregistrement de signal (12) ou des seconds moyens de détection (16) et est de préférence de l'ordre de 20 à 30 Hz.

10. Dispositif selon l'une quelconque des revendications 1 à 9, **caractérisé en ce que** les premiers moyens de détection (14) comprennent une première unité de limitation de bruit (40), qui est conçue pour supprimer le traitement ultérieur du signal de mesure à l'intérieur d'une première durée de suppression de bruit prédéfinissable de préférence de l'ordre de 125 ms après chaque valeur du signal de mesure, qui aboutit à un premier signal de marqueur (donc redéclenchable).

11. Dispositif selon l'une quelconque des revendications 1 à 10, **caractérisé en ce que** les seconds moyens de détection (16) comprennent une seconde unité de délimitation de bruit (52), qui est conçue pour supprimer le traitement ultérieur du signal de mesure en l'espace d'une seconde durée de suppression de bruit prédéfinissable de 50 ms de préférence après chaque valeur du signal de mesure, qui aboutit à un second signal de marqueur.

12. Dispositif selon l'une quelconque des revendications 1 à 11, **caractérisé en ce que** les premiers moyens de détection (14) comprennent un premier générateur de temps réfractaire (42) pour une première période réfractaire, qui est conçu pour déclencher, après chaque valeur du signal disposé en dehors de la première période réfractaire, laquelle aboutit à un premier signal de marqueur, une période réfractaire absolue prédéfinissable, qui a de préférence un ordre de grandeur de 150 ms, et **en ce que** les premiers moyens de détection (14) sont conçus pour supprimer le retraitement ultérieur du signal de mesure pendant la période réfractaire absolue.

13. Dispositif selon la revendication 12, **caractérisé en ce que** le premier générateur de période réfractaire (42) est conçu pour dissocier une période réfractaire globale qui est supérieure à la période réfractaire absolue, inclut celle-ci et est de préférence de l'ordre de 300 ms, et **en ce que** les premiers moyens de détection (14) sont conçus pour supprimer le traitement ultérieur des premiers signaux de marqueur situés à l'intérieur de la période réfractaire.

14. Dispositif selon la revendication 12 ou 13, **caractérisé en ce que** les seconds moyens de détection (16) comprennent un second générateur de période réfractaire (52) pour une seconde période réfractaire qui est conçue pour déclencher, après chaque valeur de signal située en dehors de la seconde période réfractaire, qui aboutit à un second signal de marqueur, une période réfractaire prédéfinissable qui est inférieure ou égale à la période réfractaire des premiers moyens de détection (14), et **en ce que** les seconds moyens de détection (16) sont conçus pour supprimer le traitement ultérieur de seconds signaux de marqueur dans les limites de la période réfractaire.

15. Dispositif selon l'une quelconque des revendications 10 à 14, **caractérisé par** des moyens de détection de flanc, qui sont reliés à la première et/ou la seconde unité de suppression de bruit (40, 52) et/ou le premier et/ou le second générateur de période réfractaire (42, 54) et sont conçus pour détecter un flanc de signal croissant du signal de mesure, qui est attribué à une valeur de signal de mesure qui est attribuée à un premier ou à un second signal de marqueur de telle sorte que la période de suppression de bruit respective et/ou la période réfractaire respective commence avec le signal de flanc détecté.

16. Dispositif selon la revendication 15, **caractérisé en ce que** les moyens de détection de flanc sont reliés à la première et/ou à la seconde mémoires de valeur seuil et sont conçus pour détecter un flanc de signal positif par un passage par la valeur seuil croissante du signal de mesure.

17. Dispositif selon l'une quelconque des revendications 1 à 16, **caractérisé en ce que** l'unité d'analyse (18) est conçue pour la prise en charge corrélé dans le temps de séquences de premiers et de seconds signaux de marqueur et comprend une unité de logique (58), qui est conçue pour former à partir des deux séquences une séquence de sortie unique des signaux de marqueur, qui contient aussi bien tous les premiers que tous les seconds signaux de marqueur classés dans le temps en fonction de leur attribution aux valeurs de signal de mesure entraînant le déclenchement, des premiers et des seconds signaux de mesure étant groupés en l'espace d'un temps de coïncidence prédéfinissable de 50 ms de préférence avant et/ou après un premier signal de marqueur pour former un signal de marqueur de la séquence de sortie.

18. Dispositif selon la revendication 17, **caractérisé en ce que** l'unité d'analyse (18) comprend une unité de filtre qui est reliée à l'unité de logique et est conçue pour former une séquence filtrée de signaux de marqueur de telle sorte que la séquence regroupée ne contient que des signaux de marqueur qui se situent à l'extérieur d'un temps d'élimination de préférence de 300 ms, qui commence avec chaque signal de marqueur à partir de la séquence de seconds signaux de marqueur.

19. Dispositif selon l'une quelconque des revendications 1 à 16, **caractérisé en ce que** l'unité d'analyse (18) est conçue pour la prise en charge corrélée dans le temps de séquences de premiers et seconds signaux de marqueur et
- comprend des moyens de reconnaissance de coïncidence (62), qui sont conçus pour éditer un signal de coïncidence lorsque l'un des premiers signaux de marqueur tombe en dehors de la période réfractaire et l'un des seconds signaux de marqueur tombe en dehors de la seconde durée de suppression de bruit dans une période de coïncidence de 50 ms de préférence et
- des moyens d'élimination (64), qui sont reliés aux moyens de reconnaissance de coïncidence et sont conçus pour éliminer les seconds signaux de marqueur pour le traitement ultérieur du signal qui suivent en l'espace d'un premier temps d'élimination de 300 ms de préférence un second signal de marqueur qui a abouti à un signal de coïncidence, et
- des moyens de transfert de coïncidence (66), qui sont reliés aux moyens de reconnaissance de coïncidence (62) et sont conçus pour ajouter le premier des premiers signaux de marqueur, qui suit un signal de coïncidence en dehors de la première période de suppression de bruit et à l'intérieur d'une durée de transfert de 300 ms de préférence, en conservant le contexte de temps de la séquence des seconds signaux de marqueur et pour former ainsi une séquence modifiée de signaux de marqueur,
- des moyens d'élimination de transfert de coïncidence (68), qui sont reliés aux moyens de transfert de coïncidence (66) et sont conçus pour supprimer de la séquence modifiée de signaux de marqueur tous les signaux de marqueur qui suivent en l'espace d'un second temps d'élimination de 300 ms de préférence un signal de marqueur ajouté par les moyens de transfert de coïncidence, afin de former ainsi une séquence assemblée de signaux de marqueur.

20. Dispositif selon la revendication 18 ou 19, **caractérisé en ce que** l'unité d'analyse (18) comprend une unité d'identification (60), qui
- comprend des moyens de détermination d'intervalle, qui sont réalisés pour déterminer la durée d'intervalle de temps située entre deux signaux de marqueur voisins de la séquence filtrée de signaux de marqueur ainsi qu'une
- mémoire d'intervalle de référence pour une durée d'intervalle de référence et
- des moyens de comparaison d'intervalle qui sont reliés aux moyens de détermination d'intervalle et à la mémoire d'intervalle de référence et sont conçus pour détecter les intervalles de la séquence filtrée de signaux de marqueur qui sont plus courts que la durée d'intervalle de référence,
- un compteur qui est relié aux moyens de comparaison d'intervalle et conçu pour déterminer le nombre de signaux de marqueur ou d'intervalles, situé à l'intérieur d'une période de la séquence filtrée de signaux de marqueur, entre respectivement deux signaux de marqueur voisins et le nombre de ces intervalles qui sont plus courts que la durée d'intervalle de référence, et
- un générateur de signal qui est relié à l'unité de comptage d'intervalle et à une mémoire de quotients et est conçu de telle sorte que le générateur de signal émet un signal dans le cas où la fraction des intervalles qui sont plus courts que la durée d'intervalle de référence dépasse une fraction mémorisée dans la mémoire de quotients par rapport au nombre total d'intervalles d'une partie de la séquence filtrée.

21. Dispositif selon l'une quelconque des revendications 1 à 20, **caractérisé en ce que** l'unité d'enregistrement de signal (12) présente un décimateur (26) qui balaie le signal de mesure de préférence dans un cycle de 4096 Hz et émet de préférence avec une cadence de 512 Hz une nouvelle valeur de balayage respective pour le signal de mesure.

22. Dispositif selon l'une quelconque des revendications 1 à 21, **caractérisé en ce que** l'unité d'enregistrement de signal (12) comprend un convertisseur analogique/numérique (28) qui est conçu pour convertir chaque valeur de balayage analogique, en particulier, les valeurs de sortie du décimateur (26), en une valeur numérique, de sorte que, à la sortie du convertisseur numérique/analogique, on a une séquence discrète dans le temps de valeurs numériques qui correspondent aux signaux de mesure.

23. Dispositif selon l'une quelconque des revendications 1 à 22, **caractérisé en ce que** l'unité d'enregistrement de signal (12) comprend des moyens de formation de moyenne (30) pour la formation d'une moyenne mobile du signal de mesure.

24. Dispositif selon l'une quelconque des revendications 1 à 23, **caractérisé en ce que** les premiers de détection (14) comprennent un amplificateur de signal (32) avec un facteur d'amplification adaptable.

25. Dispositif selon la revendication 24, avec une unité de stimulation pour l'envoi d'impulsions appropriées à la stimulation cardiaque, **caractérisé en ce que** l'amplificateur de signal est conçu pour augmenter le facteur d'amplification dans le cas où l'unité de stimulation émet une impulsion et/ou la séquence des seconds signaux de marqueur présente un signal de marqueur à l'intérieur d'une période prédéfinie de 200 ms de préférence avant l'envoi d'une impulsion.

26. Dispositif selon la revendication 25, **caractérisé par** une unité d'activation qui est reliée aux seconds moyens de détection et à l'amplificateur de signal de telle sorte qu'il active les seconds moyens de détection dans les cas où l'amplificateur de signal augmente le facteur d'amplification.

27. Dispositif selon l'une quelconque des revendications 1 à 25, **caractérisé par** une unité d'activation (56), qui est reliée aux premiers et aux seconds moyens de détection de telle sorte qu'il active les seconds moyens de détection lorsque la séquence des premiers signaux de marqueur dépasse une fréquence prédéfinie de 140 signaux de marqueur de préférence par minute.

28. Dispositif selon l'une quelconque des revendications 1 à 27, **caractérisé en ce que** les seconds moyens de détection comprennent une mémoire de signal qui est reliée par un convertisseur analogique/numérique à l'unité d'enregistrement de signal et pour la mémorisation d'au moins une partie respectivement actuelle du signal de mesure.
